(19) Europäisches Patentamt
European Patent Office
Office européen des brevets

(11) EP 2 425 249 B1

(12) **FASCICULE DE BREVET EUROPEEN**

(45) Date de publication et mention de la délivrance du brevet:
**16.07.2014 Bulletin 2014/29**

(21) Numéro de dépôt: **10727062.1**

(22) Date de dépôt: **29.04.2010**

(51) Int Cl.:
***G01N 33/542*** *(2006.01)* ***G01N 33/94*** *(2006.01)*

(86) Numéro de dépôt international:
**PCT/FR2010/050814**

(87) Numéro de publication internationale:
**WO 2010/125314 (04.11.2010 Gazette 2010/44)**

(54) **PROCEDE DE DETECTION DE COMPOSES MODULATEURS DE DIMERES DE PROTEINES MEMBRANAIRES A DOMAINE VFT**

VERFAHREN ZUM NACHWEIS VON DIMERE VON VFT-DOMÄNE-MEMBRANPROTEINEN MODULIERENDEN VERBINDUNGEN

METHOD FOR DETECTING COMPOUNDS MODULATING DIMERS OF VFT DOMAIN MEMBRANE PROTEINS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **30.04.2009 FR 0952908**

(43) Date de publication de la demande:
**07.03.2012 Bulletin 2012/10**

(73) Titulaires:
- **CISBIO BIOASSAYS**
  **30200 Codolet (FR)**
- **Centre National de la Recherche Scientifique**
  **75016 Paris (FR)**

(72) Inventeurs:
- **DOUMAZANE, Etienne**
  **F-34090 Montpellier (FR)**
- **ZWIER, Jurriaan**
  **F-30650 Rochefort Du Gard (FR)**
- **TRINQUET, Eric**
  **F-30130 Pont Saint Esprit (FR)**
- **PIN, Jean-Philippe**
  **F-34000 Montpellier (FR)**

(74) Mandataire: **Nevant, Marc et al**
**Cabinet Beau de Loménie**
**158, rue de l'Université**
**75340 Paris Cedex 07 (FR)**

(56) Documents cités:
**EP-A1- 1 865 316** **WO-A1-2005/033709**
**WO-A2-2007/026099** **US-B1- 6 824 990**

- **MAUREL D ET AL: "Cell-surface protein-protein interaction analysis with time-resolved FRET and snap-tag technologies: Application to GPCR oligomerization" NATURE METHODS 200806 GB, vol. 5, no. 6, juin 2008 (2008-06), pages 561-567, XP002556321 cité dans la demande**
- **LIU JIANFENG ET AL: "Molecular determinants involved in the allosteric control of agonist affinity in the GABAB receptor by the GABAB2 subunit." THE JOURNAL OF BIOLOGICAL CHEMISTRY 16 APR 2004, vol. 279, no. 16, 16 avril 2004 (2004-04-16), pages 15824-15830, XP002556320 ISSN: 0021-9258**
- **PIN J-P ET AL: "The activation mechanism of class-C G-protein coupled receptors." BIOLOGY OF THE CELL / UNDER THE AUSPICES OF THE EUROPEAN CELL BIOLOGY ORGANIZATION JUN 2004, vol. 96, no. 5, juin 2004 (2004-06), pages 335-342, XP002556319 ISSN: 0248-4900**
- **IMBERT PIERRE-ELOI ET AL: "Recommendations for the reduction of compound artifacts in time-resolved fluorescence resonance energy transfer assays." ASSAY AND DRUG DEVELOPMENT TECHNOLOGIES JUN 2007 LNKD- PUBMED: 17638536, vol. 5, no. 3, juin 2007 (2007-06), pages 363-372, XP002593112 ISSN: 1540-658X**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

## ETAT DE LA TECHNIQUE

L'invention est relative aux composés modulateurs de récepteurs membranaires ayant un intérêt dans la recherche de nouveaux médicaments, de nouveaux modulateurs du goût et dans l'agriculture.

Les récepteurs couplés aux protéines G (RCPG) constituent la plus grande famille de récepteurs membranaires des mammifères puisqu'elle représente 3,4 % du génome. L'isolation, puis le clonage des RCPG ont permis d'identifier chez l'homme autour de 900 gènes, dont environ 500 correspondent aux récepteurs olfactifs et gustatifs et 400 à des récepteurs capables de lier des ligands endogènes. Ainsi, l'hétérogénéité de ces récepteurs assure une reconnaissance très large de signaux tant externes (odeurs, lumière, molécules du goût) qu'internes (hormones et neurotransmetteurs).

Les RCPG sont référencés en trois classes majeures :

La classe A, avec comme archétype la rhodopsine, est la classe la plus représentée. Le site de liaison des ligands de ces récepteurs implique principalement les domaines transmembranaires ainsi que les boucles extracellulaires des RCPG.

La classe B des RCPG se caractérise par un large domaine N-terminal extracellulaire (entre 100 et 500 acides aminés) contenant des cystéines conservées et engagées dans des ponts disulfures. Les ligands de ces récepteurs se lient exclusivement au niveau des domaines extracellulaires (domaine N-terminal et boucles extracellulaires des RCPG).

Les récepteurs de classe C sont caractérisés par un très grand domaine extracellulaire (~600 acides aminés) ressemblant aux protéines périplasmiques bactériennes, impliquées dans le transport d'acides aminés, de sucres et d'ions, appelé domaine VFT (acronyme de l'expression anglaise « Venus Flytrap »). Ils comprennent les récepteurs métabotropiques du glutamate (récepteurs mGluR1, mGluR2, mGluR3, mGluR4, mGluR5, mGluR6, mGluR7, mGluR8), le récepteur de l'acide gamma-aminobutyrique ou GABA (récepteur $GABA_B$), le récepteur du goût sucré et le récepteur du goût *umami,* le récepteur sensible au calcium extracellulaire (CaSR) ainsi qu'un récepteur des acides aminés basiques (GPRC6a). D'autres RCPG de classe C à domaine VFT existent dans le monde animal, notamment les récepteurs V2R des phéromones.

Il est communément admis que tous les RCPG de classe C à domaine VFT sont dimériques, c'est-à-dire qu'ils sont composés de deux sous-unités et que l'association des deux sous-unités est essentielle au fonctionnement du récepteur. Certains sont homodimériques, constitués de deux sous-unités identiques (les récepteurs mGluR, CaSR et GPRC6A) alors que les autres sont hétérodimériques, constitués de sous-unités issues de l'expression de gènes distincts (le récepteur $GABA_B$ avec les deux sous-unités GB1 et GB2, le récepteur du goût sucré avec TAS1R2 et TAS1R3, le récepteur du goût *umami,* avec TAS1R1 et TAS1R3).

Dans le cas des RCPG de classe C à domaine VFT, chaque sous-unité est dotée, dans sa partie N-terminale, d'un large domaine extracellulaire, responsable de la fixation du ligand orthostérique, et appelé domaine « Venus-FlyTrap » ou domaine VFT. Les domaines VFT sont des domaines protéiques conservés, présents dans les RCPGs de classe C, ainsi que dans quelques autres récepteurs notamment les récepteurs ionotropes du glutamate communément appelés récepteurs AMPA, NMDA ou Kaïnate, les récepteurs de la famille du récepteur du peptide natriurétique atrial (NPR-A et NPR-B) et le récepteur Venus-Kinase (VKR) qui est présent chez les insectes.

Les domaines VFT de ces récepteurs partagent une structure caractéristique en forme de coquillage, avec deux lobes organisés autour d'une charnière et ménageant une crevasse dans laquelle se fixe le ligand orthostérique. La flexibilité de la charnière permet une fermeture des deux lobes en réponse à la fixation du ligand activateur. La fermeture du domaine VFT par le ligand orthostérique est la première étape essentielle à l'activation du récepteur. Inversement, des ligands antagonistes maintiennent le domaine VFT en position ouverte et le récepteur dans un état inactivé.

Au sein d'un récepteur dimérique, les domaines VFT des deux sous-unités s'organisent en dimère de domaines VFT, comme l'illustre la structure cristallographique aux rayons X obtenue sur le dimère de domaines VFT du récepteur mGluR1 (Kunishima et al., Nature. 2000 Oct 26;407(6807):971-7.; Tsuchiya et al., Proc Natl Acad Sci U S A. 2002 Mar 5;99(5):2660-5). Le modèle d'activation des RCPG de classe C à domaine VFT suggère que la fixation d'un ligand activateur provoque non seulement la fermeture d'au moins un VFT, mais également un changement d'orientation d'un VFT par rapport à l'autre. Ce mouvement relatif conduit à un rapprochement des deux extrémités C-terminales des VFT qui serait lui-même responsable du changement de conformation des domaines membranaires nécessaire au recrutement de la protéine G et à la transduction du signal.

Les RCPG de classe C à domaine VFT sont des cibles extrêmement importantes pour la recherche de nouveaux médicaments. Les récepteurs mGluR sont impliqués notamment dans la maladie de Parkinson, la schizophrénie, la douleur ; le récepteur $GABA_B$ joue un rôle dans l'épilepsie et les phénomènes de dépendance aux drogues ; le récepteur

CaSR est impliqué dans l'ostéoporose. Ces récepteurs sont également intéressants pour la recherche de nouvelles molécules au goût sucré (récepteur du goût sucré) ou bien de nouveaux exhausteurs de goût (récepteur du goût *umami*). Enfin, la recherche de modulateurs des récepteurs aux phéromones V2R est intéressante dans le domaine de l'agriculture.

Il existe des techniques de criblage permettant de suivre la liaison d'un ligand et/ou l'activation du RCPG consécutive à la liaison du ligand : GTP-gammaS (radioactif), élecrophysiologie, utilisation de ligands radioactifs. Ces méthodes de criblage ne peuvent pas être utilisées à grande échelle.

Les plateformes de criblage à haut débit utilisent généralement des cellules de mammifères, transfectées de manière stable ou transitoire pour exprimer le récepteur d'intérêt. L'action d'un composé à tester est identifiée par des tests fonctionnels basés sur la détection de variation de la concentration intracellulaire de messagers secondaires apparaissant dans la cellule après l'activation de protéines G couplées audit récepteur. Le messager secondaire mesuré dépend du type de protéine G couplée au récepteur d'intérêt :

- Récepteurs couplés aux protéines Gs : l'accumulation du second messager AMPc est mesurée pour caractériser l'action pharmacologique sur un RCPG couplé à une protéine Gs.
- Récepteurs couplés aux protéines Gq (c'est le cas des récepteurs du glutamate mGluR1, mGluR5, du récepteur du calcium extracellulaire CaSR, du récepteur des acides aminés basiques GPRC6A) : l'accumulation d'un métabolite de l'IP3, par exemple l'IP1. Alternativement, en chargeant les cellules avec une sonde calcique fluorescente, il est possible de révéler l'augmentation transitoire de la concentration calcique intracellulaire.
- Récepteurs couplés aux protéines Gi (exemples : les récepteurs du glutamate mGluR2, mGluR3, mGluR4, mGluR6, mGluR7, mGluR8, le récepteur du GABA $GABA_B$, les récepteurs du goût sucré et du goût umami) : de façon indirecte, on stimule le récepteur par la forskoline ou l'isoprotérénol, qui provoque une augmentation de la concentration en AMPc. L'inhibition de cette augmentation par un composé testé est représentative du couplage du récepteur avec la protéine Gi. Une autre possibilité, généralement préférée pour le criblage, est de co-exprimer le récepteur d'intérêt avec une protéine G chimérique, issue de la fusion d'une partie de la protéine Gi, capable de reconnaître le RCPG dans son état activé, et d'une partie de la protéine Gq, capable de déclencher la voie IP3/Ca2+. Dans ces conditions, l'activation du récepteur peut être décelée par le signal transitoire calcique qui survient.

Les techniques existantes sont mal adaptées à la mise en évidence de composés capables de moduler l'activation des RCPG de classe C dimériques : les effets agonistes sont très difficiles à détecter lorsque les récepteurs ont une faible affinité pour leur ligand naturel (c'est le cas pour les récepteurs mGlu); les tests fonctionnels basés sur la mesure d'un message secondaire résultant de la transduction du signal à l'intérieur de la cellule (AMPc, IP3, IP1), ne sont pas non plus appropriés au regard de la difficulté relative de leur mise en oeuvre et de la complexité des voies effectrices couplées à des récepteurs dimériques. Les techniques de FRET (transfert d'énergie de résonance de Förster) basées sur l'expression de RCPG fusionnés au niveau de leurs domaines intracellulaires (C-terminal) avec des protéines fluorescentes ne sont pas non plus adaptées en raison du bruit de fond important provenant des dimères présents dans les compartiments intracellulaires.

Le brevet américain US 6,824,990 décrit de manière générale des méthodes d'étude de la dimérisation des RCPG, basées sur le marquage de RCPG susceptibles de former des dimères par des composés donneurs et accepteurs d'énergie, en particulier des protéines fluorescentes, et la mesure de variation de FRET. Ce brevet ne décrit pas de solution technique permettant de sélectionner des composés modulateurs de dimères de RCPG de classe C. Par ailleurs, il est essentiellement illustré par l'utilisation de protéines fluorescentes qui sont peu appropriées au cas présent en raison du bruit de fond important généré par les dimères présents dans les différents compartiments intracellulaires.

La demande WO 2007/026099 décrit un procédé pour la mise en évidence d'un processus biologique par mesure d'un signal de FRET, procédé qui comprend une étape d'incorporation, dans un milieu de mesure comprenant une membrane lipidique, de deux entités biologiques chacune marquée par un des membres d'un couple de partenaires de FRET.

Maurel et al (Nat. Methods ; Juin 2008 ; 5(6):561-7) ont appliqué la technique de FRET à l'étude de dimères de récepteur du GABA : Ils ont marqué chacune des sous-unités $GABA_{B1}$ et $GABA_{B2}$ du récepteur $GABA_B$ par un cryptate de d'europium (EuPyridine-bis-bipyridine) et un fluorophore accepteur (d2). Ces études n'ont pas permis d'observer de variation du signal de FRET lorsque le récepteur est activé par son agoniste, le GABA, et suggèrent que cette approche n'est pas adaptée à l'étude de ces dimères.

La présente invention se propose de fournir un procédé pour la mise en évidence de composés ayant un effet modulateur sur l'état d'activation des dimères de protéines membranaires à domaine VFT, en particulier des RCPG de classe C, permettant de déterminer si des composés à tester ont un effet pharmacologique sur ces récepteurs.

Cette invention revêt une importance particulière dans la mesure où il n'existe pas à ce jour de techniques permettant de cribler efficacement et facilement ce type de récepteurs dimériques pour trouver de nouveaux médicaments.

## DESCRIPTION DES FIGURES

La figure 1 représente la variation du signal de FRET émis par un dimère mGluR2-mGluR2 en fonction de la concentration en glutamate.
La figure 2 représente la variation du signal de FRET émis par un dimère mGluR2-mGluR2 en fonction de la concentration en antagoniste LY341495 et en présence ou en l'absence de glutamate.
La figure 3 représente la variation du signal de FRET émis par un dimère mGluR2-mGluR2 en fonction de la concentration en glutamate ou en DCG-IV (agoniste partiel).
La figure 4 représente la variation du signal de FRET émis par un dimère mGluR2-mGluR2 en fonction de la concentration de glutamate en présence ou en l'absence de 4-MPPTS (modulateur allostérique positif).
La figure 5 représente la variation du signal de FRET émis par un dimère mGluR2-mGluR2 (marquage Snaptag sur chaque sous-unité) en fonction de la concentration en glutamate ou en DGC-IV.
La figure 6 représente la variation du signal de FRET émis par les dimères mGluR2-mGluR2, mGluR3-mGluR3 et mGluR4-mGluR4, en fonction de la concentration en glutamate.
La figure 7 représente la variation du signal de FRET émis par un dimère mGluR3-mGluR3 en fonction de la concentration en LY341495.
La figure 8 représente la variation du signal de FRET émis par les dimères mGluR2-mGluR2, mGluR3-mGluR3, mGluR4-mGluR4, mGluR2-mGluR3 et mGluR2-mGluR4 en fonction de la concentration en glutamate.
La figure 9 représente la variation du ratio de FRET émis par les dimères mGluR1-mGluR1, mGluR2-mGluR2, mGluR3-mGluR3 et mGluR8-mGluR8 en présence de glutamate ou de LY341495, ou en l'absence de ces composés.
La figure 10 représente la variation du ratio de FRET émis par les dimères mGluR1-mGluR1, mGluR2-mGluR2, mGluR3-mGluR3, mGluR4-mGluR4, mGluR5-mGluR5 et mGluR8-mGluR8, en présence de glutamate ou de LY341495, ou en l'absence de ces composés.
La figure 11 représente la variation du ratio de FRET émis par un dimère mGluR8-mGluR8, en fonction de la fenêtre temporelle, en présence de glutamate ou de LY341495, ou en l'absence de ces composés.
La figure 12 représente la variation du ratio de FRET émis par les dimères mGluR8-mGluR8 et mGluR2-mGluR2, en fonction de la fenêtre temporelle, en présence de glutamate ou de LY341495, ou en l'absence de ces composés.
La figure 13A représente un dimère de mGluR2, chacun conjugué à l'enzyme Snaptag (S), et comportant la mutation F756P qui empêche leur couplage avec les protéines G.
La figure 13B représente un dimère de mGluR2, chacun conjugué à l'enzyme Snaptag (S), et comportant la mutation F756P qui empêche leur couplage avec les protéines G, ainsi que la mutation C234A qui rend ces protéines insensibles aux modulateurs allostériques.
La figure 13C représente un dimère de mGluR2, chacun tronqué à partir de l'acide aminé 597 (milieu de la première boucle intracellulaire i1), fusionné à l'enzyme Snaptag (S), et ne comportant donc qu'un seul domaine transmembranaire, un domaine CRD et un domaine VFT.
La figure 13D représente un dimère de mGluR2, chacun tronqué après l'acide aminé 561 (ne comportant donc qu'un domaine VFT et un domaine CRD), fusionné à l'enzyme Snaptag (S), et auxquels ont été insérés après le domaine riche en cystéine (CRD, càd. après les acides aminés LPQEY), un peptide signal d'une ancre GPI (RRSSSTVLFSSPVILLISFLIFLIVG - SEQ ID NO :7).
La figure 13E représente un dimère de mGluR2, chacun tronqué à partir de l'acide aminé 499 (ne comportant donc qu'un domaine VFT), fusionné à l'enzyme Snaptag (S), et auxquels ont été insérés après le domaine VFT (i.e. après les acides aminés GPLPAS), un peptide signal d'une ancre GPI (RRSSSTVLFSSPVILLISFLIFLIVG - SEQ ID NO :7).
La figure 13F représente un dimère de récepteurs chimériques comportant chacun (i) les 561 premiers acides aminés de mGluR2 (domaine VFT + CRD, se terminant après les acides aminés LPQEY), fusionné à l'enzyme Snaptag (S), et (ii) les 414 premiers acides aminés (sans la méthionine 1) du variant court du récepteur dopaminergique D2, correspondant à son domaine transmembranaire.
La figure 14 représente la variation du signal de FRET en fonction du temps.

## DESCRIPTION

L'invention est relative à un procédé de sélection de composés ayant un effet modulateur sur l'état d'activation d'un dimère de protéines à domaine VFT exprimées dans des membranes cellulaires présentes dans un milieu de mesure, ledit dimère étant constitué d'une première protéine et d'une seconde protéine, lesdites protéines étant identiques ou différentes, le procédé comprenant les étapes suivantes :

(a) marquage de la première et de la seconde protéine dans la partie N-terminale de leurs domaines VFT par les

membres d'un couple de partenaires de FRET;
(b) excitation des partenaires de FRET et mesure du signal de FRET en l'absence et en présence d'un composé à tester ;
(c) sélection du composé à tester comme composé modulateur si une différence de signal de FRET en l'absence et en présence d'un composé à tester est mesurée à l'étape (b).

le signal de FRET étant mesuré à l'étape (b) dans une fenêtre temporelle dans laquelle les signaux mesurés en présence et en l'absence d'un composé agoniste ou antagoniste de référence, sont différents.

De manière surprenante, les inventeurs ont mis en évidence que l'absence de variation de signal de FRET en présence d'un modulateur de l'activité de dimères de protéines à domaine VFT, observée dans l'art antérieur par Maurel et al, est liée au choix de la fenêtre temporelle dans laquelle le signal est mesuré. Les signaux de FRET sont classiquement mesurés après un délai suivant l'excitation lumineuse du milieu de mesure, et pendant une durée donnée (le temps d'intégration). Les produits et instruments disponibles pour effectuer des dosages biologiques basés sur la mesure de signaux de FRET sont conçus pour éviter à l'utilisateur de faire varier cette fenêtre temporelle de mesure du signal.

Dans le cas des dimères de protéines à domaine VFT, les inventeurs ont découvert que lorsque l'on mesure la décroissance du signal de FRET au cours du temps en présence ou en l'absence d'un composé agoniste de référence, on observe un point d'inversion correspondant au temps après excitation auquel les valeurs de FRET en présence et en l'absence de ce composé agoniste de référence sont identiques.

Les inventeurs ont déterminé qu'avant ce point d'inversion, le signal de FRET en l'absence d'un composé agoniste de référence (courbe en gris dans la figure 14) était supérieur à celui mesuré en présence d'agoniste (courbe noire dans la figure 14). A partir du point d'inversion, le signal de FRET observé en l'absence d'agoniste devient au contraire inférieur à celui mesuré en présence d'agoniste.

Par ailleurs, si le signal est mesuré dans certaines fenêtres temporelles comprenant le point d'inversion (par exemple f1, cf. figure 14), aucune variation du signal de FRET en présence et en l'absence de composé modulateur n'est observée. En d'autres termes, les inventeurs ont identifié le problème expliquant l'absence de variation de signal de FRET observée dans l'art antérieur, à savoir l'existence d'un point d'inversion dans la fenêtre temporelle utilisée par le passé.

Il découle de cette découverte que lorsque le signal de FRET est mesuré dans une fenêtre temporelle située avant ou après ce point d'inversion (f2, f4, cf. figure 14), les signaux de FRET mesurés en présence et en l'absence de composé agoniste de référence seront toujours différents. Il est possible de choisir une fenêtre temporelle comprenant le point d'inversion (f3, cf. figure 14), mais dans ce cas, il faudra s'assurer que les signaux mesurés en présence et en l'absence d'un composé agoniste ou antagoniste de référence sont différents.

L'invention consiste donc en un procédé de sélection de composés ayant un effet modulateur sur l'état d'activation d'un dimère de protéines à domaine VFT exprimées dans des membranes cellulaires présentes dans un milieu de mesure, ledit dimère étant constitué d'une première protéine et d'une seconde protéine, lesdites protéines étant identiques ou différentes, le procédé comprenant les étapes suivantes :

(a) marquage de la première et de la seconde protéine dans la partie N-terminale de leurs domaines VFT par les membres d'un couple de partenaires de FRET;
(b) excitation des partenaires de FRET et mesure du signal de FRET en l'absence et en présence d'un composé à tester ;
(c) sélection du composé à tester comme composé modulateur si une différence de signal de FRET en l'absence et en présence d'un composé à tester est mesurée à l'étape (b),

le signal de FRET étant mesuré à l'étape (b) dans une fenêtre temporelle dans laquelle les signaux mesurés en présence et en l'absence d'un composé agoniste ou antagoniste de référence, sont différents.

On considère que les signaux mesurés en présence et en l'absence d'un composé agoniste ou antagoniste de référence sont différents si l'une des conditions suivantes est remplie :

- s'il existe une différence de plus de 20% entre les valeurs moyennes des signaux en présence et en l'absence de composé agoniste de référence, ou bien
- si les valeurs moyennes des signaux en présence et en l'absence de composé agoniste diffèrent de plus de 3 fois l'écart type le plus important (soit l'écart type du signal mesuré en l'absence d'agoniste de référence, soit celui du signal mesuré en présence de ce composé).

La fenêtre temporelle peut être déterminée expérimentalement en faisant varier le délai de mesure du signal après excitation et la durée pendant laquelle ce signal est mesuré (temps d'intégration). Ces paramètres sont facilement modifiables avec les fluorimètres disponibles dans le commerce.

Dans une mise en oeuvre particulière, le procédé selon l'invention est caractérisé en ce que le signal mesuré à l'étape (b) l'est avec un délai compris entre 180 et 800 ps après l'excitation et un temps d'intégration de 200 à 2000 $\mu$s.

Dans une autre mise en oeuvre particulière, le procédé selon l'invention est caractérisé en ce que :

(i) soit le rayon de Förster ($R_0$) dudit couple de partenaires de FRET est compris entre 20 et 55 Å, et le signal mesuré à l'étape (b) l'est avec un délai compris entre 20 et 100 $\mu$s après l'excitation et un temps d'intégration de 100 à 500 $\mu$s ;
(ii) soit le rayon de Förster ($R_0$) dudit couple est supérieur à 55 Å, et le signal mesuré à l'étape (b) l'est avec un délai compris entre 180 et 800 $\mu$s après l'excitation et un temps d'intégration de 200 à 2000 $\mu$s.

Le procédé de l'invention permet, de manière relativement simple, de cribler de larges banques de composés pour évaluer leurs effets sur les protéines membranaires à domaine VFT, et en particulier les RCPG de classe C à domaine VFT qui représentent une cible de choix notamment pour la découverte de nouveaux médicaments.

Le procédé selon l'invention peut ainsi être mis en oeuvre pour découvrir de nouveaux modulateurs des récepteurs métabotropiques du glutamate (constitués de dimères de sous-unités choisies parmi : mGluR1, mGluR2, mGluR3, mGluR4, mGluR5, mGluR6, mGluR7, mGluR8), du récepteur de l'acide gamma-aminobutyrique ou GABA (récepteur $GABA_B$ constitué d'une sous-unité GB1 et d'une sous-unité GB2), des récepteurs liés à la perception du goût sucré (dimère TAS1R1-TAS1R3) ou du goût umami (dimère TAS1R2 TAS1R3), du récepteur sensible au calcium extracellulaire (homodimère de sous-unités CaSR) et du récepteur des acides aminés basiques (homodimère de sous-unités GPRC6a). Ces termes englobent les formes sauvages de ces récepteurs, ainsi que des formes mutantes, tronquées, chimériques ou modifiées tels que décrites ci-après.

Le procédé selon l'invention est préférentiellement mis en oeuvre avec des récepteurs métabotropiques du glutamate, ou avec des récepteurs du goût.

Lorsque le procédé selon l'invention est mis en oeuvre sur les récepteurs du glutamate, ces derniers peuvent être constitués des dimères suivants : mGluR1-mGluR1; mGluR1-mGluR2; mGluR1-mGluR3; mGluR1-mGlu4; mGluR1-mGluR5; mGluR1-mGluR6; mGluR1-mGluR7; mGluR2-mGluR2; mGluR2-mGluR3; mGluR2-mGluR4; mGluR2-mGluR5; mGluR2-mGluR6; mGluR2-mGluR7; mGluR3-mGluR3; mGluR3-mGluR4; mGluR3-mGluR5; mGluR3-mGluR6; mGluR3-mGluR7; mGluR4-mGluR4; mGluR4-mGluR5; mGluR4-mGluR6; mGluR4-mGluR7; mGluR5-mGluR5; mGluR5-mGluR6; mGluR5-mGluR7; mGluR6-mGluR6; -mGluR6-mGluR7; mGluR7-mGluR7 ; mGlur8-mGluR1; mGlur8-mGluR2; mGlur8-mGluR3; mGlur8-mGlu4; mGlur8-mGluR5; mGlur8-mGluR6; mGlur8-mGluR7 ; mGlur8-mGluR8. Dans une mise en oeuvre particulièrement préférée, le dimère est choisi parmi : un homodimère de mGluR1, un homodimère de mGluR2, un homodimère de mGluR3, un homodimère de mGluR4, un homodimère de mGluR5, un homodimère de mGluR8, un hétérodimère mGluR2-mGluR3, un hétérodimère mGluR2-mGluR4.

En ce qui concerne les récepteurs du goût, le dimère peut être choisi parmi les dimères : TAS1R2-TAS1R3 ou TAS1R1-TAS1R3.

Le procédé selon l'invention peut être mis en oeuvre avec des dimères de protéines correspondant à des RCPG tronqués, par exemple des protéines tronquées dans la première boucle intracellulaire et ne comportant donc qu'un seul domaine transmembranaire (figure 13C). Les variants d'épissages de protéines à domaines VFT ne comportant pas de domaines transmembranaires (Ferraguti et al, Cell Tissue Res. 2006 Nov;326(2):483-504) sont également utilisables, sous réserve que lorsque un tel variant constitue l'une des sous-unités du dimère étudié, l'autre sous-unités comporte au moins un domaine transmembranaire.

Le procédé selon l'invention peut également être mis en oeuvre avec des récepteurs chimériques comprenant un domaine extracellulaire d'un récepteur à domaine VFT, et un ou plusieurs domaines transmembranaires d'autres récepteurs (voir Figure 13F). De tels récepteurs chimériques peuvent être produits par les techniques classiques de biologie moléculaire consistant à fabriquer des vecteurs d'expression comportant l'ADN codant pour les domaines en question, dont les séquences nucléiques sont disponibles par exemple dans la base de donnée Genbank, et à transfecter ces vecteurs dans des cellules. Malitschek et al (Mol Pharmacol. 1999 Aug;56(2):448-54.) ont décrit de tels récepteurs chimériques.

Le procédé selon l'invention peut également être mis en oeuvre avec des constructions protéiques comprenant un domaine extracellulaire de récepteur à domaine VFT et une ancre glycosylphosphatidylinositol (« ancre GPI ») à la place d'un domaine transmembranaire, qui va permettre d'ancrer la partie protéique à la surface de la membrane plasmique (voir figure 13D et 13E). De telles protéines et leur fabrication sont décrites en particulier par Liu et al (J Biol Chem. 2004 Apr 16;279(16):15824-3).

Dans une autre mise en oeuvre particulière de l'invention, les dimères utilisés sont des RCPG mutants qui ne sont couplés à aucune voie de signalisation intracellulaire. Ces RCPG mutants sont connus, et comprennent par exemple des RCPG comportant une ou plusieurs mutations empêchant le couplage avec les protéines G, tel que le mutant F756P de mGluR2 (voir Figure 13A) Cette mise en oeuvre est préférée lorsque les dimères sont exprimés par la cellule de manière stable, ce qui peut avoir un effet toxique pour la cellule.

Il peut aussi être intéressant d'utiliser des dimères constitués de RCPG comportant une mutation les rendant

insensibles à l'effet des modulateurs allostériques, comme par exemple le mutant C234A de mGluR2 (Figure 13B). L'utilisation de tels mutants pour mettre en oeuvre l'invention permets de découvrir des composés modulateurs qui ne sont pas des modulateur allostériques.

Enfin, le procédé selon l'invention peut également être mis en oeuvre avec d'autres récepteurs à domaines VFT qui ne sont pas des RCPG de classe C, en particulier les récepteurs ionotropes du glutamate (communément appelés récepteurs AMPA, NMDA ou Kaïnate), les récepteurs de la famille du récepteur du peptide natriurétique atrial (NPR-A et NPR-B) et le récepteur Venus-Kinase (VKR) qui est présent chez les insectes.

*Définitions :*

*« Couple de partenaires de FRET » :* cette expression désigne un couple constitué d'un composé fluorescent donneur d'énergie (ci-après « composé fluorescent donneur ») et d'un composé accepteur d'énergie (ci-après « composé accepteur »); lorsqu'ils sont à proximité l'un de l'autre et lorsqu'ils sont excités à la longueur d'onde d'excitation du composé fluorescent donneur, ces composés émettent un signal de FRET. Il est connu que pour que deux composés fluorescents soient partenaires de FRET, le spectre d'émission du composé fluorescent donneur doit recouvrir partiellement le spectre d'excitation du composé accepteur.

*« Signal de FRET » :* désigne tout signal mesurable représentatif d'un FRET entre un composé fluorescent donneur et un composé accepteur. Un signal de FRET peut donc être une variation de l'intensité ou de la durée de vie de luminescence du composé fluorescent donneur ou du composé accepteur lorsque ce dernier est fluorescent.

*« Fenêtre temporelle » :* désigne la période de temps qui commence lorsque le signal de FRET est mesuré après un délai suivant l'excitation lumineuse du milieu de mesure et se termine à la fin du temps d'intégration. Par exemple, une fenêtre temporelle « 50-450 $\mu$s » signifie que le signal de FRET est mesuré au bout d'un délai de 50 $\mu$s après l'excitation lumineuse du milieu de mesure, pendant une durée (temps d'intégration) de 400 $\mu$s.

*« Milieu de mesure » :* désigne le contenu du puits d'une plaque, d'un tube à essai ou de tout autre contenant approprié au mélange de cellules ou de membranes cellulaires avec les réactifs nécessaires à la mise en oeuvre de l'invention.

*« Composé Modulateur » :* par composés ayant un effet modulateur sur l'état d'activation de dimères de protéines à domaine VFT, on entend des composés ayant un effet activateur ou désactivateur.

Les composés activateurs peuvent être en particulier des composés agonistes, des agonistes partiels ou des modulateurs allostériques positifs. L'effet des composés modulateurs allostériques positifs n'est visible que sur des dimères activés par un de leurs agonistes de référence : dans ce cas, les modulateurs allostériques positifs vont provoquer une augmentation de l'activation du récepteur. En l'absence d'agoniste de référence, l'effet du modulateur allostérique positif n'est pas visible.

Les composés désactivateurs diminuent l'activité des dimères et sont soit des agonistes inverses, dans le cas où ils ont un effet de désactivation sur un dimère constitutivement actif, soit des antagonistes ou des modulateurs allostériques négatifs lorsqu'ils entraînent la désactivation du dimère préalablement activé par un de ses agonistes de référence.

Les composés modulateurs sont ajoutés au milieu de mesure et leur effet sur l'état d'activation est observé selon le procédé de l'invention. Il n'y pas de limitation quant à la nature chimique de ces composés modulateurs ni quant à leur mode d'action : l'invention permet en effet de détecter des changements de conformation au niveau des domaines VFT, ces changements résultant de la liaison des composés modulateurs à l'une, l'autre ou aux deux sous unités constitutives du dimère, mais également à d'autres protéines membranaires qui vont elles-mêmes exercer un effet sur le dimère à domaines VFT d'intérêt.

Selon une mise en oeuvre particulière, le procédé selon l'invention comprend les étapes suivantes :

(a) marquage de la première et de la seconde protéine dans la partie N-terminale de leurs domaines VFT par les membres d'un couple de partenaires de FRET, le rayon de Förster (R0) dudit couple étant compris entre 20 et 55 Å ;
(b) mesure du signal de FRET en l'absence et en présence de composé à tester ;
(c) sélection du composé à tester comme composé modulateur par comparaison de la variation du signal de FRET en présence ou en l'absence dudit composé à tester avec celle mesurée en présence ou en l'absence d'un composé modulateur connu dudit dimère.

Dans cette mise en oeuvre particulière, le signal de FRET est avantageusement mesuré à l'étape (b) avec un délai de 50 $\mu$s après l'excitation et un temps d'intégration de 450 $\mu$s.

Selon la nature du modulateur connu du dimère et la variation du signal de FRET mesuré en présence ou en l'absence du composé à tester par rapport à celle observée en présence ou en l'absence dudit composé modulateur connu, on détermine la nature du composé à tester.

Ainsi, si le modulateur connu du dimère est un agoniste, le composé à tester sera un composé activateur si la

variation du signal de FRET mesuré en présence et en l'absence dudit composé à tester va dans le même sens que celle observée en présence et en l'absence d'un composé agoniste connu, et un composé désactivateur si ladite variation du signal de FRET va dans le sens opposé à celle observée en présence et en l'absence dudit composé agoniste connu ;

De même, si le modulateur du dimère est un antagoniste connu dudit dimère, le composé à tester sera un composé activateur si la variation du signal de FRET mesuré en présence et en l'absence de composé à tester va dans le sens opposé à celle observée en présence et en l'absence dudit composé antagoniste connu, et un composé désactivateur si ladite variation du signal de FRET va dans le même sens que celle observée en présence et en l'absence dudit composé antagoniste connu.

En pratique, on peut comparer les courbes doses-réponses du composé à tester avec celles obtenues avec un composé agoniste ou antagoniste connu.

Il est à noter que dans certains cas, les mesures de signal de FRET avec ou sans composé à tester peuvent être effectuées en présence d'une quantité donnée d'un composé agoniste de référence. C'est en particulier le cas lorsque l'on recherche des modulateurs allostériques positifs, négatifs ou des antagonistes. Cela n'est bien sûr pas nécessaire lorsque l'on recherche des composés agonistes ou agonistes partiels.

Dans le cas particulier de la mise en oeuvre du procédé selon l'invention avec des dimères de récepteurs métabotropiques du glutamate (mGluR), et lorsque le signal est mesuré avec un délai de 50µs après excitation et un temps d'intégration de 450µs, les inventeurs ont découvert que :

- l'ajout dans le milieu de mesure d'un composé agoniste du dimère de mGluR étudié provoque une diminution du signal de FRET émis, par rapport à celui observé en l'absence de ce composé; dans le cas d'un agoniste partiel, c'est-à-dire dont l'effet est moins important que celui de l'agoniste « naturel », cette diminution est d'une moindre intensité.
- l'ajout dans le milieu de mesure d'un composé antagoniste du dimère de mGluR étudié provoque une augmentation du signal de FRET émis, par rapport à celui observé en l'absence de ce composé. Cet effet peut être observé sur des dimères constitutivement actifs ou bien sur des dimères activés par un composé agoniste.
- l'ajout dans le milieu de mesure d'un modulateur à effet allostérique positif provoque une diminution du signal de FRET émis, par rapport à celui observé en l'absence d'un tel modulateur. Cet effet ne peut être observé qu'en présence d'agoniste dans le milieu de mesure, par exemple l'agoniste naturel du récepteur étudié.
- l'ajout dans le milieu de mesure d'un modulateur à effet allostérique négatif provoque une augmentation du signal de FRET émis, par rapport à celui observé en l'absence d'un tel modulateur. Cet effet ne peut être observé qu'en présence d'agoniste dans le milieu de mesure par exemple l'agoniste naturel du récepteur étudié.

A l'inverse, lorsque le signal est mesuré avec un délai plus long, par exemple avec un délai de 500 µs après l'excitation, et un temps d'intégration de 500 µs, les inventeurs ont découvert que le signal mesuré après ajout dans le milieu de mesure d'un composé agoniste du dimère de mGluR était plus important que celui mesuré en l'absence de ce composé.

Il est important de noter que le procédé selon l'invention permet également de mettre en évidence des composés ayant un effet transrégulateur, c'est-à-dire des composés qui ne se lient pas sur le dimère de protéines à domaine VFT mais à un autre récepteur membranaire qui va lui-même interagir avec le dimère en question : le récepteur mGluR2 par exemple peut être régulé par le récepteur de la sérotonine 5HT2A (Gonzàlez-Maeso et al, Nature, Vol. 452, Mars 2008 93-99) et le procédé selon l'invention permet de mettre en évidence ce type de modulation du dimère mGluR2.

Le procédé selon l'invention permet donc de mettre en évidence des composés qui ne se lient pas au dimère étudié mais à une autre protéine membranaire qui joue un rôle de régulation (activation ou inhibition) sur ledit récepteur. On peut ainsi mettre en évidence des composés ayant un effet activateur ou désactivateur indirect sur ledit récepteur.

**<u>Membranes Cellulaires comportant des dimères de protéines à domaine VFT</u>**

Le procédé selon l'invention est mis en oeuvre dans un milieu de mesure contenant des membranes cellulaires dans lesquelles se trouvent les dimères de protéines à domaine VFT. Le milieu de mesure comprend donc, lors de l'ajout des composés partenaires de FRET, soit des cellules intactes vivantes, adhérentes ou en suspension, soit des préparations de membranes cellulaires en suspension, obtenues par exemple par lyse des cellules et centrifugation fractionnée. Les techniques de préparation de suspensions de membranes sont connues de l'homme du métier. Le procédé selon l'invention est mis en oeuvre de manière préféré sur des cellules intactes vivantes.

Les dimères de protéines transmembranaires à domaine VFT sont exprimés dans les membranes cellulaires de manière naturelle par les cellules, ou bien sont exprimés en utilisant les techniques classiques de biologie moléculaire, via des vecteurs d'expression introduits dans les cellules de manière stable ou transitoire. Les réactifs destinés à la production d'ADN hétérologue dans des cellules, de manière stable ou transitoire, sont disponibles dans le commerce et les séquences d'ADN codant pour les protéines transmembranaires à domaine VFT, en particulier celles codant pour

les RCPG de classe C cités plus haut sont disponibles dans les bases de données telles que Genbank. Comme indiqué plus haut, lorsque les dimères sont exprimés par les cellules de manière stable, il est conseillé d'utiliser des RCPG mutants qui ne sont couplés à aucune protéine G, afin d'éviter les éventuels effets toxiques pour la cellule.

**Le couple de partenaires de FRET**

L'une des caractéristiques essentielles de l'invention est que chacune des protéines constitutives du dimère étudié est marquée par un membre d'un couple de partenaires de FRET.

Dans une mise en oeuvre préférée du procédé selon l'invention, le rayon de Fôrster ($R_0$) du couple de partenaires de FRET est compris entre 20 et 55 Å. Dans une autre mise en oeuvre préférée, ce rayon est supérieur à 55 Å,

L'homme du métier est capable de choisir parmi les composés partenaires de FRET disponibles dans le commerce, ceux dont le rayon de Förster est compris dans ces plages. La théorie de Förster enseigne en effet comment déterminer la valeur de $R_0$ pour un couple de partenaires de FRET donné.

Selon cette théorie, le FRET est défini comme un transfert d'énergie non radiatif résultant d'une interaction dipôle- dipôle entre un donneur et un accepteur d'énergie. Ce phénomène physique nécessite une compatibilité énergétique entre ces molécules. Cela signifie que le spectre d'émission du donneur doit recouvrir, au moins partiellement, le spectre d'absorption de l'accepteur. Ce recouvrement des spectres est défini par l'intégrale de recouvrement J(λ) :

$$J_{(\lambda)} = \int f_D(\lambda)\varepsilon_A(\lambda)\lambda^4 d\lambda$$

où $f_D$ est l'intensité de la fluorescence émise par le donneur à une longueur d'onde donnée et $\varepsilon_A$ le coefficient d'extinction molaire de l'accepteur. Le facteur J reflète donc la capacité d'une paire de fluorophores à émettre et absorber de l'énergie à la même longueur d'onde.

En accord avec la théorie de Förster, le FRET est un processus qui dépend de la distance séparant les deux molécules, donneur et accepteur, comme le montre la formule suivante :

$$E = \frac{1}{1 + (R/R_0)^6}$$

où R est la distance effective qui sépare les deux molécules et $R_0$ le rayon de Förster. Ce dernier correspond à la distance donneur/accepteur pour laquelle l'efficacité du transfert d'énergie est de 50%. L'expression mathématique pour le calcul de cette distance s'écrit :

$$R_0 = (10^{-3}\, \kappa^2\, n^{-4} Q_D\, J)^{1/6} \times 9730$$

où J est l'intégrale de recouvrement, n l'indice de réfraction du milieu ($n^{-4}$ est généralement compris entre 1/3 et 1/5), $Q_D$ le rendement quantique du donneur en l'absence d'accepteur et $k^2$ le facteur d'orientation qui est fonction de l'orientation relative des dipôles du donneur et de l'accepteur. Même si la valeur de $k^2$ est théoriquement comprise entre 0 et 4, 2/3 est la valeur habituellement utilisée pour déterminer le $R_0$. En effet, $k^2$ est assimilé à 2/3 lorsque le donneur et l'accepteur présentent un degré de liberté suffisant pour être aléatoirement orientés dans l'espace. Cette condition est généralement satisfaite pour les fluorophores attachés à des biomolécules car ils peuvent avoir une certaine liberté de rotation.

Un exemple de calcul de $R_0$ pour le couple cryptate d'europium Pyridine-bisbipyridine et d2 (un fluorophore commercialisé par la société Cisbio Bioassay) est détaillé par Maurel et al (Nature methods 2008, supplementary figures and texts). Dans cet exemple le $R_0$ est de 65,5 Å.

De nombreux composés donneurs ou accepteurs d'énergie ont été décrits et sont disponibles dans le commerce.

Les composés fluorescents peuvent être choisis dans le groupe suivant : les allophycocyanines, en particulier celle connue sous la dénomination commerciale XL665 ; les molécules organiques luminescentes, telles que les rhodamines, les cyanines, les squaraines, les coumarines, les proflavines, les acridines, les fluorescéines, les fluorophores connus sous la dénomination Bodipy, les fluorophores connus sous la dénomination Atto, les fluorophores connus sous la dénomination Dy, les composés connus sous la dénomination AlexaFluor, le nitrobenzoxadiazole les complexes métalliques fluorescents, tels que les cryptates de terre rare, les chélates de terre rare (en particulier les chélates et

cryptates d'europium, de terbium, de samarium, de dysprosium, de neodymium); les particules inorganiques luminescentes comme les nanocristaux («quantum dots» en langue anglaise»). Ces composés fluorescents peuvent être utilisés soit comme composés fluorescents donneurs soit comme composés fluorescents accepteurs dans un système de FRET.

L'utilisation de protéines fluorescentes est possible en tant que composé accepteur. L'utilisation de ces protéines en tant que donneur n'est en revanche pas conseillée dans la mesure où ces protéines provoqueraient une augmentation significative du bruit de fond provenant des différents compartiments cellulaires dans lesquels ces protéines sont exprimées avant leur adressage à la membrane plasmique.

Les protéines fluorescentes suivantes peuvent être utilisées selon l'invention : les protéines fluorescentes cyans (AmCyan1, Midori-Ishi Cyan, mTFP1), les protéines fluorescentes vertes (EGFP, AcGFP, TurboGFP, Emerald, Azami Green, ZsGreen), les protéines fluorescentes jaunes (EYFP, Topaz, Venus, mCitrine, YPet, PhiYFP, ZsYellow1, mBanana), les protéines fluorescentes oranges et rouges (Orange kusibari, mOrange, tdtomato, DsRed, DsRed2, DsRes-Express, DsRed-Monomer, mTangerine, AsRed2, mRFP1, JRed, mCherry, mStrawberry, HcRed1, mRaspberry, HcRed-Tandem, mPlim, AQ143), les protéines fluorescentes dans le rouge lointain (mKate, mKate2, tdKatushka2).

Les composés donneurs d'énergie à durée de vie longue (>0,1 ms, de préférence comprise entre 0,5 et 6 ms), en particulier les chélates ou cryptates de terre rare sont avantageux puisqu'ils permettent d'effectuer des mesures en temps résolu, c'est-à-dire de mesurer des signaux de TR-FRET en s'affranchissant d'une grande partie du bruit de fond émis par le milieu de mesure. Ils sont pour cette raison et de manière générale préférés pour la mise en oeuvre du procédé selon l'invention. Les chélates ou cryptates d'europium ou de terbium en particulier sont particulièrement appropriés en tant que membre du couple de FRET donneur d'énergie.

Les complexes de dysprosium (Dy3+), de samarium (Sm3+), de neodymium (Nd3+), d'ytterbium (Yb3+) ou encore d'erbium (Er3+) sont des complexes de terres rares appropriés aux fins de l'invention, les complexes d'europium (Eu3+) et de terbium (Tb3+) étant particulièrement préférés.

De très nombreux complexes de terres rares ont été décrits et plusieurs sont actuellement exploités commercialement notamment par les sociétés PerkinElmer, Invitrogen et Cisbio Bioassay.

Des exemples de chélates ou cryptates de terres rares appropriés aux fins de l'invention sont :

- Les cryptates de terres rares, comportant un ou plusieurs motifs pyridine. De tels cryptates de terre rare sont décrits par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de terres rares sont commercialisés par la société Cisbio Bioassay. On peut citer à titre d'exemple non limitatif les cryptates d'europium de formules ci-dessous (qui peuvent être couplés au composé à marquer via un groupe réactif, ici par exemple un groupe NHS) :

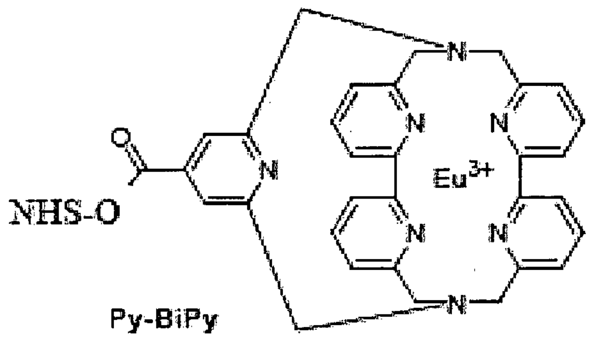

Py-BiPy

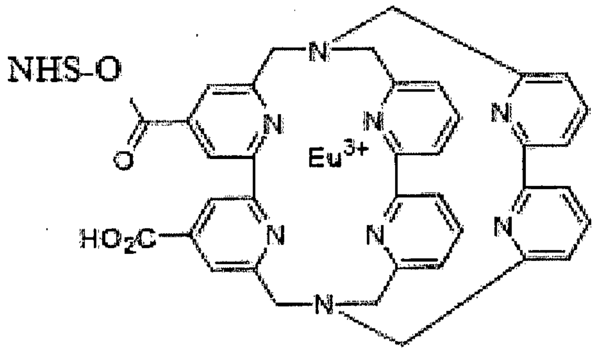

TrisBiPy-Eu

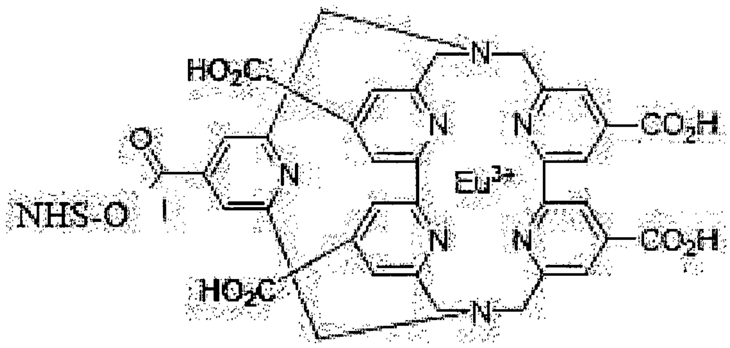

K-Py-BiPy-tetraacide-Eu

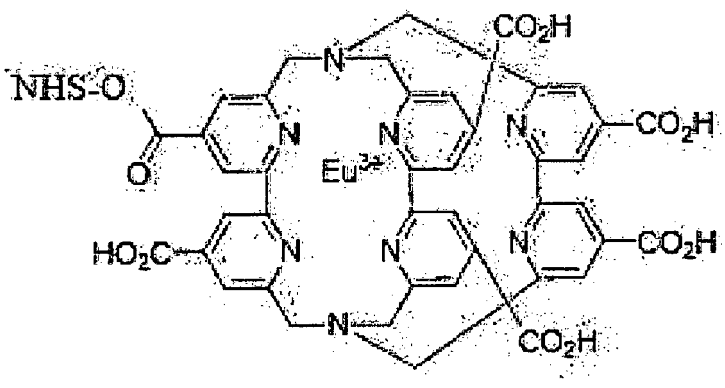

K-TrisBiPy-pentaacide-Eu

Le cryptate d'europium Py-BiPy-tétraacide-Eu est particulièrement adapté à la mise en oeuvre de l'invention en raison de ses propriétés de résistance à l'extinction de fluorescence dans les milieux biologiques.

- Les chélates de terres rares décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates composés d'un ligand nonadenté tel que la terpyridine. Ces chélates de terres rares sont commercialisés par la société PerkinElmer.

- Des complexes de terres rares constitués d'un agent chélatant tel que le tetraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes

suitable for usage in cellulo", peuvent également être utilisés. On peut aussi utiliser les complexes décrits dans la demande WO2009/10580.

- Le cryptate de terbium Tb(KR) de formule ci-dessous (qui peut être couplé au composé à marquer via un groupe réactif, ici par exemple un groupe NHS) :

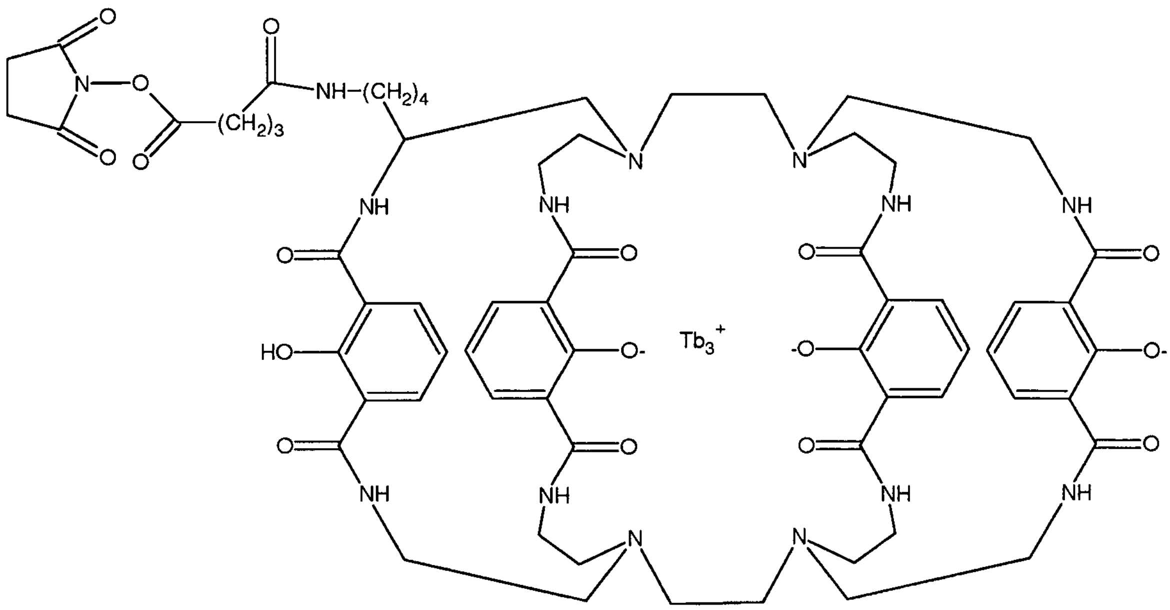

et dont la synthèse est décrite dans la demande internationale WO2008063721 est l'un des cryptates de terbium les plus appropriés à la mise en oeuvre de l'invention

- Le cryptate de Terbium Lumi4-Tb de la société Lumiphore, commercialisé par Cisbio bioassays.

- Le « quantum dye » de la société Research Organics, de formule ci-dessous (qui peut être couplé au composé à marquer via un groupe réactif, ici NCS) :

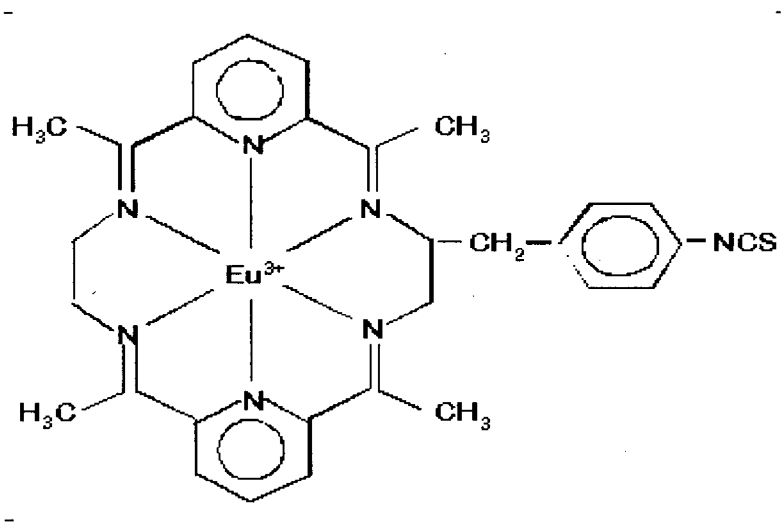

- Le chélate de terbium DTPA-cs124 Tb, commercialisé par la société Invitrogen de formule ci-dessous (qui peut être couplé au composé à marquer via un groupe réactif R) et dont la synthèse est décrite dans le brevet américain US 5,622,821.

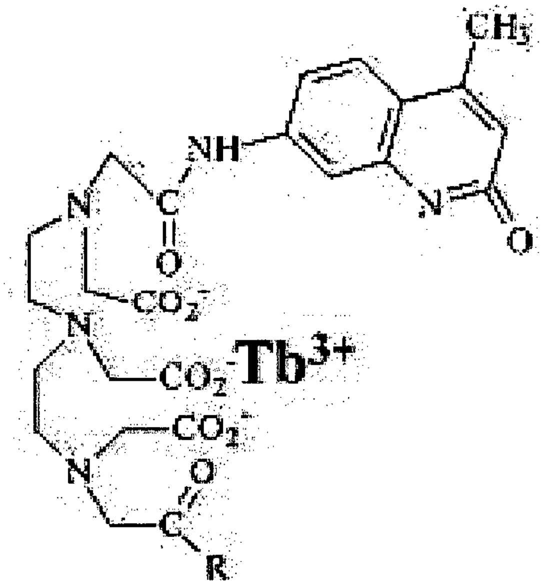

En fonction du donneur choisi, et en appliquant les principes de la théorie de Förster rappelée précédemment, l'homme du métier est à même de déterminer le rayon de Förster du couple de partenaire de FRET à utiliser selon l'invention.

A titre d'exemples non limitatifs, le tableau ci-après liste des couples de partenaires de FRET dont le rayon de Förster est compris entre 20 et 55 Å:

| | Tb(KR) | DTPA-cs124 Tb | Lumi4-Tb |
|---|---|---|---|
| **Atto425** | 21 Å | 21 Å | 21 Å |
| **Alexa430** | 21 Å | 21 Å | 21 Å |
| **Coumarine343** | 23 Å | 23 Å | 23 Å |
| **Orange d'acridine** | 26 Å | 26 Å | 26 Å |
| **Jaune lucifer** | 26 Å | 26 Å | 26 Å |
| **Jaune acridine** | 27 Å | 27 Å | 27 Å |
| **proflavine** | 29 Å | 29 Å | 29 Å |
| **Atto465** | 30 Å | 30 Å | 30 Å |
| **nitrobenzoxadi azole** | 33 Å | 33 Å | 33 Å |
| **Coumarine 6** | 34 Å | 34 Å | 34 Å |
| **Alexa 750** | 40 Å | 40 Å | 40 Å |
| **Cy7** | 40 Å | 40 Å | 40 Å |
| **Fluorescéine** | 46 Å | 46 Å | 46 Å |
| **Rouge du nil** | 46 Å | 46 Å | 46 Å |
| **Alexa488** | 46 Å | 46 Å | 46 Å |
| **Dy495** | 46 Å | 46 Å | 46 Å |
| **Dy490** | 46 Å | 46 Å | 46 Å |
| **Vert oregon** | 47 Å | 47 Å | 47 Å |
| **Atto488** | 47 Å | 47 Å | 47 Å |
| **Atto495** | 47 Å | 47 Å | 47 Å |
| **Alexa514** | 47 Å | 47 Å | 47 Å |

(suite)

| | Tb(KR) | DTPA-cs124 Tb | Lumi4-Tb |
|---|---|---|---|
| **Atto520** | 48 Å | 48 Å | 48 Å |
| **Cy2** | 52 Å | 52 Å | 52 Å |
| **Rhodamine6G** | 53 Å | 53 Å | 53 Å |
| **Alexa700** | 54 Å | 54 Å | 54 Å |
| **Alexa680** | 55 Å | 55 Å | 55 Å |
| **Atto532** | 55 Å | 55 Å | 55 Å |
| **Alexa532** | 55 Å | 55 Å | 55 Å |
| **EGFP** | 45 Å | - | 45 Å |
| **YFP** | 43 Å | - | 43 Å |
| **mPlum** | 53 Å | - | 53 Å |

Parmi ces couples, les couples Tb (KR) / fluorésceine, Lumi4-Tb / Fluorescéine sont préférés.

| | Py-BiPy- tetraacide-Eu | TrisBiPy-Eu |
|---|---|---|
| **Rhodamine6G** | 20 Å | 22 Å |
| **Tétraméthylrhodamine** | 38 Å | 43 Å |
| **Sulforhodamine 101** | 45 Å | 55 Å |
| **Merocyanine 540** | 31 Å | 41 Å |
| **Atto565** | 36 Å | 45 Å |
| **Cy3** | 26 Å | 24 Å |
| **Atto550** | 26 Å | 34 Å |
| **Cy3.5** | 48 Å | 41 Å |
| **Dy547** | 25 Å <$R_0$<35 Å | - |
| **Dy548** | 25 Å <$R_0$<35 Å | - |
| **Dy549** | 25 Å <$R_0$<35 Å | - |
| **Dy554** | 25 Å <$R_0$<35 Å | - |
| **Dy555** | 25 Å <$R_0$<35 Å | - |
| **Dy556** | 25 Å <$R_0$<35 Å | - |
| **Dy560** | 25 Å <$R_0$<35 Å | - |
| **mStrawberry** | 44 Å | 52 Å |
| **mCherrry** | 51 Å | -- |

Les composés Alexa et vert oregon sont commercialisés par la société Invitrogen; les composé Atto sont commercialisés par la société Attotec ; les composés Dy sont commercialisés par la société Dyomics ; Les composés Cy sont commercialisés par la société Amersham Biosciences ; les autres composés sont commercialisés par divers fournisseurs de réactifs chimiques, tels que les sociétés Sigma, Aldrich ou Acros.

Parmi les couples précédents, les couples dont les R0 sont compris entre 21 et 48 sont préférés aux fins de l'invention.

A titre d'exemples non limitatifs, le tableau ci-après liste des couples de partenaires de FRET dont le rayon de Förster est supérieur à 55 Å, et qui nécessite donc une lecture du signal de FRET avec un délai compris entre 180 et 800 μs après l'excitation et un temps d'intégration de 200 à 2000 μs.

| | Tb(KR) | DTPA-cs124 Tb | Lumi4-Tb |
|---|---|---|---|
| **Dy648** | 58 Å | 58 Å | 58 Å |
| **Dy647** | 58 Å | 58 Å | 58 Å |
| **tetraméthylrhodamine** | 56 Å | 56 Å | 56 Å |
| **Sulfrhodamine 101** | 58 Å | 58 Å | 58 Å |
| **Merocyanine 540** | 58 Å | 58 Å | 58 Å |
| **Atto565** | 60 Å | 60 Å | 60 Å |
| **Cy3** | 65 Å | 65 Å | 65 Â |
| **Cy5** | 59 Å | 59 Å | 59 Å |
| **Atto590** | 61 Å | 61 Å | 61 Å |
| **Atto550** | 62 Å | 62 Å | 62 Å |
| **Cy3.5** | 63 Å | 63 Å | 63 Å |
| **Cy5.5** | 58 Å | 58 Å | 58 Å |
| **Dy547** | >60 Å | >60 Å | >60 Å |
| **Dy548** | >60 Å | >60 Å | >60 Å |
| **Dy549** | >60 Å | >60 Å | >60 Å |
| **Dy554** | >60 Å | >60 Å | >60 Å |
| **Dy555** | >60 Å | >60 Å | >60 Å |
| **Dy556** | >60 Å | >60 Å | >60 Å |
| **Dy560** | >60 Å | >60 Å | >60 Å |
| **Alexa647** | 58 Å | 58 Å | 58 Å |
| **mCherry** | 58 Å | - | 58 Å |
| **mStrawberry** | 60 Å | - | 60 Å |

| | Py-BiPy-tetraacide- Eu | TrisBiPy-Eu |
|---|---|---|
| **Alexa680** | 71 Å | 73 Å |
| **Alexa700** | 76 Å | 81 Å |
| **Alexa750** | 59 Å | 62 Å |
| **Alexa647** | 65 Å | 65 Å |
| **Cy5** | 66 Å | 64 Å |
| **Cys.5** | 71 Å | 72 Å |
| **Cy7** | 59 Å | 64 Å |
| **Dy647** | 65 Å | 65 Å |
| **Dy648** | 65 Å | 65 Å |
| **Atto590** | 59 Å | 62 Å |
| **mCherry** | - | 56 Å |

Les couples Tb(KR) / Dy648, Lumi4-Tb / Dy648, Tb(KR) / Dy647 et Lumi4-Tb / Dy647 sont particulièrement préférés.

**Marquage des protéines transmembranaires par les membres d'un couple de partenaires de FRET**

Une autre des caractéristiques essentielles de l'invention réside dans le fait que le marquage des protéines transmembranaires à domaine VFT par un donneur ou un accepteur d'énergie est effectué en position N-terminale des domaines VFT de chacune des deux sous-unités constituant le dimère.

Plusieurs techniques peuvent être utilisées pour marquer la protéine à domaine VFT avec un donneur ou un accepteur, en particulier on peut utiliser l'une quelconque des techniques ci-après :

*(a) Couplage de la protéines à domaine VFT avec un donneur ou un accepteur de manière indirecte (non covalente)*

Le donneur ou l'accepteur peut être couplé avec la protéine à domaine VFT par l'intermédiaire d'un couple de partenaires de liaison dont l'un au moins est de nature protéique. Dans cette approche, la protéine à domaine VFT est fusionnée avec le partenaire de liaison de nature protéique par les techniques classiques de biologie moléculaire (construction d'un vecteur d'expression comprenant une séquence de nucléotides codant pour la protéine à domaine VFT, fusionnée avec celle codant pour le partenaire de liaison protéique, et introduction du vecteur d'expression dans la cellule). Selon l'invention, le partenaire de liaison est présent dans la partie N-terminale de la protéine à domaine VFT, de préférence à son extrémité.

Le donneur ou l'accepteur est conjugué de manière covalente à l'autre partenaire de liaison, que l'on appelle ici agent de couplage, qui sera ensuite ajouté au milieu extracellulaire. La reconnaissance des partenaires de liaison permet le marquage indirect de la protéine à domaine VFT par le donneur ou l'accepteur.

A titre d'exemples non limitatifs de partenaires de liaison particulièrement adaptés à la mise en oeuvre de l'invention on peut citer :

- le couple constitué de la séquence cystéine-cystéine-X-X-cystéine-cystéine (SEQ ID N°1) dans laquelle X est un acide aminé quelconque et d'un composé bi-arsenic. Ces composés bi-arsenic peuvent être facilement marqués avec une molécule organique du type fluorescéine ou rhodamine (voir B.A.Griffin et al. (1998) Science. 1998, 281, 269-271 et S.A. Adams et al. (2002) J. Am. Chem. Soc. 2002, 124, 6063-6076 pour des détails sur la technologie).
- Le peptide BTX (bungarotoxine), composé de 13 acides aminés qui est reconnu par la bungarotoxine (BTX), peut être couplé à une molécule fluorescente (voir C. M. McCann et al. (2005), Biotechnique (2005), 38, 945-952).
- Le couple streptavidine (ou avidine) / Biotine : La séquence de liaison de la Streptavidine (SBP-Tag) est une séquence formée par 38 acides aminés qui présente une haute affinité pour la biotine pouvant être préalablement marquée avec un donneur ou un accepteur (voir C. M. McCann et al. (2005), Biotechnique (2005), 38, 945-952).
- La séquence de l'enzyme dihydrofolate réductase d'E. coli (eDHFR) qui lie spécifiquement et avec une haute affinité des ligands, tels que la triméthoprime sur lesquels peuvent être greffés le donneur ou l'accepteur selon la technologie dénommée « Ligand link Universal labelling technology » de la Société Active Motif.
- Les couples tags/antitags sont des partenaires de liaison fréquemment utilisés pour marquer des protéines. Le terme « tag » désigne une petite « étiquette » protéique constituée d'un séquences d'acides aminés, généralement mais pas obligatoirement assez courte (moins de 15 acides aminés), qui est fusionnée à la protéine à domaine VFT ou bien est naturellement présente dans cette protéine. Le terme « antitag » désigne un anticorps se liant de manière spécifique audit « tag ». Dans cette mise en oeuvre, l'anticorps « antitag » est lié de manière covalente au donneur ou à l'accepteur. Lorsque l'anticorps ainsi marqué est ajouté au milieu extracellulaire, il se lie au « tag » conjugué à la protéine à domaine VFT et l'interaction « tag/antitag » permet le marquage indirect de cette protéine par le donneur ou l'accepteur.

A titre d'exemple non-limitatif de couples « tags/antitags », on peut citer les couples suivants dont les membres sont disponibles commercialement :

GST/anticorps anti-GST dans lequel GST représente la glutatione S-transférase ou un de ses fragments ; 6HIS/anticorps anti-6HIS dans lequel 6HIS est un peptide constitué de 6 histidines ; Myc/anticorps anti-Myc dans lequel Myc est un peptide constitué des acides aminés 410-419 de la protéine Myc humaine ;
FLAG/anticorps anti-FLAG dans lequel FLAG est un peptide ayant les 8 acides aminés DYKDDDDK (SEQ ID N°2) ;
HA/anticorps anti HA dans lequel HA est un épitope de l'hémaglutinine d'Influenza, constitué des 9 acides aminés YPYDVPFYA (SEQ ID N°3). Il est clair que la nature exacte du tag n'est pas critique pour la mise en oeuvre de l'invention.

*(b) Couplage de la protéine à domaine VFT avec un donneur ou un accepteur de manière directe (covalente)*

Dans cette approche, le donneur ou l'accepteur est couplé à la protéine à domaine VFT par une liaison

covalente ; plusieurs techniques ont été décrites et les réactifs nécessaires à leur mise en oeuvre sont disponibles commercialement. Pour ce couplage, on pourra utiliser l'une quelconque des techniques ci-après :

- formation d'une liaison covalente au niveau d'un groupe réactif présent sur la protéine à domaine VFT, en particulier au niveau d'un des groupes suivants : le groupe amino terminal, les groupes carboxylates des acides aspartiques et glutamiques, les groupes amines des lysines, les groupes guanidines des arginines, les groupes thiol des cystéines, les groupes phénols des tyrosines, les cycles indoles des tryptophanes, les groupes thioéthers des méthionines, les groupes imidazoles des histidines.
  Ces groupes présents sur la protéine à domaine VFT peuvent former une liaison covalente avec un groupe réactif porté par le donneur ou l'accepteur. Les groupes réactifs appropriés sont connus de l'homme du métier : un donneur ou un accepteur fonctionnalisé par un groupe maléimide sera par exemple capable de se lier de manière covalente avec les groupes thiols portés par les cystéines de la protéine. De même, un donneur/accepteur portant un ester de N-hydroxysuccinimide sera capable de se fixer de manière covalente à une amine de la protéine à domaine VFT.
- Utilisation d'une enzyme suicide
  Les enzymes suicides sont des protéines qui ont une activité enzymatique modifiée par des mutations spécifiques qui leur confèrent la capacité de lier rapidement et de façon covalente un substrat. Ces enzymes sont dites suicides car chacune ne peut lier qu'une seule molécule fluorescente, l'activité de l'enzyme étant bloquée par la fixation du substrat. Ces enzymes constituent par conséquent un outil de choix pour marquer spécifiquement des protéines d'intérêt avec un rapport d'une molécule fluorescente pour une protéine. On peut citer à titre d'exemple non limitatif les enzymes suivantes :

  - les mutants de l'O6-alkylguanine DNA alkyltransférase (AGT). Les enzymes SNAP-tag (Juillerat et al, Chemistry & biology, Vol.10, 313-317 Avril 2003) et CLIP-tag (Gautier et al, Chemistry et Biology, 15, 128-136, février 2008) commercialisées par la société NEB sont des mutants de l'AGT humaine dont les substrats sont respectivement, l'O6-benzylguanine (ci-après abrégée BG) et l'O2-benzylcytosine (ci-après abrégée BC). L'enzyme N-AGT (Gronemeyer et al (Protein engineering, design & selection, vol. 19, no 7, pp 309-3016, 2006) est un autre mutant de cette enzyme dont la réactivité avec l'O6-benzylguanine est meilleure que celle de l'enzyme SNAP-tag. Les mutants de l'O6-alkylguanine DNA alkyltransférase sont les enzymes suicides préférées.
  - les mutants d'une déshalogénase (telle que l'enzyme HaloTag commercialisée par Promega) qui génèrent également une réaction enzymatique du type suicide (voir WO 04/072232 A2), dont certains des substrats sont des composés de la famille des chloroalcanes, en particulier les chloroalcanes comportant le motif $-NH-CH_2CH_2-O-CH_2CH_2-O-(CH_2)_6-Cl$. Dans ce cas, le donneur/accepteur sera conjugué à ce type de motif.
  - la protéine ACP (protéine de transport d'acyles, « Acyl Carrier Protein » en langue anglaise), sur laquelle est transféré, en présence de phosphopanthéthéine transférase, le résidu 4'-phosphopantéthéine du coenzyme A sur une sérine de l'ACP (N.George et al, Journal of the American Chemical society 126 (2004) p 8896-8897). Lorsque cette approche est utilisée pour marquer la protéine à domaine VFT par le donneur ou l'accepteur, il est nécessaire de rajouter au milieu réactionnel de la phosphopantéthéine transférase. La société NEB commercialise un fragment de l'ACP sous le nom commercial « ACP-Tag » pour le marquage de protéines.

Dans cette approche, une enzyme suicide est fusionnée avec la protéine à domaine VFT dans sa partie N-terminale par les techniques classiques de biologie moléculaire (fabrication d'un vecteur d'expression comprenant la séquence nucléique codant pour l'enzyme suicide et celle codant pour la protéine à domaine VFT, et introduction de ce vecteur dans la cellule), et le substrat de l'enzyme, lié de manière covalente à un donneur/accepteur, est introduit dans le milieu extracellulaire. La réaction enzymatique a pour conséquence la liaison covalente du substrat marqué à l'enzyme, et donc le marquage de la protéine à domaine VFT par le donneur ou l'accepteur.

L'utilisation d'enzymes suicides est particulièrement préférée pour la mise en oeuvre de l'invention : dans ce cas, la protéine membranaire à domaine VFT est exprimée sous forme d'une protéine de fusion comprenant la protéine à domaine VFT et, à son extrémité N-terminale, une enzyme suicide ou un fragment d'enzyme suicide capable de se lier de manière covalente avec son substrat : le marquage de la protéine à domaine VFT peut alors être effectué par addition dans le milieu de mesure d'un des composés membre du couple de partenaires de FRET, lié de manière covalente au substrat de ladite enzyme suicide.

Dans une mise en oeuvre encore plus préférée, chacune des sous-unités constituant le dimère est exprimée sous forme de protéine de fusion avec une enzyme suicide. Dans ce cas les enzymes suicides utilisées pour chaque sous-unité peuvent être différentes ou identiques.

Maurel et al ont décrit la préparation de plasmides codant pour une protéine de fusion comprenant une enzyme suicide (Snaptag) dans la partie N-terminale de la protéine à domaine VFT (GABA B1, GABA B2, mGlu1) et leur transfection dans des cellules (Nature Methods, 2008, Supplementary methods). Les méthodes utilisées relèvent des techniques générales de biologie moléculaire.

## Mesure du signal de FRET

La mesure du signal de FRET est effectuée de manière classique à l'aide d'un fluorimètre qui permet l'excitation du milieu de mesure à la longueur d'onde d'absorption du donneur d'énergie et la mesure de la luminescence émise par le milieu de mesure à la longueur d'onde d'émission du composé donneur et/ou du composé accepteur si ce dernier est fluorescent.

La luminescence émise par le milieu est détectée après un délai suivant l'excitation, et pendant une période appelée temps d'intégration, ces deux paramètres étant réglables sur les fluorimètres disponibles dans le commerce.

Dans un mode de réalisation préféré, en particulier lorsque le rayon de Förster ($R_0$) du couple de partenaires de FRET est compris dans la gamme allant de 20 Å à 55 Å, le signal est mesuré avec un délai de 20 à 100 μs après l'excitation, et un temps d'intégration de 100 à 500 μs. Les fenêtres temporelles suivantes, individuellement, sont des fenêtre préférées : 50-300 μs, 50-400 μs, 50-450 μs, 40-250 μs, 100-300 μs.

Dans un autre mode de réalisation préféré, et quel que soit le rayon de Förster ($R_0$) du couple de partenaires de FRET, le signal est mesuré avec un délai compris entre 180 et 800 μs après l'excitation et un temps d'intégration de 200 à 2000 μs. Les fenêtres temporelles suivantes, individuellement, sont des fenêtres préférées : 300-650 μs, 500-1000 μs, 500-1500 μs, 800-1800 μs. Comme mentionné plus haut, ce dernier mode de réalisation est obligatoire lorsque le rayon de Förster ($R_0$) du couple de partenaires de FRET est supérieur à 55 Å.

Le rapprochement des composés donneur et accepteur d'énergie provoque une augmentation du transfert d'énergie (augmentation du FRET) qui va se traduire par :

- une augmentation de la luminescence émise à la longueur d'onde d'émission de l'accepteur s'il est fluorescent ;
- une diminution de la luminescence émise à la longueur d'onde d'émission du donneur ; une diminution de la durée de vie de fluorescence du composé donneur.

L'évolution du signal de FRET peut donc être mesurée en détectant la luminescence émise par le donneur ou celle émise par l'accepteur. De manière préférée, le signal de FRET est mesuré en détectant la luminescence émise par le composé accepteur.

De manière encore plus préférée, les variations de FRET sont mesurées en détectant à la fois (mais pas forcément simultanément) la luminescence émise par le composé donneur à sa longueur d'onde d'émission et la luminescence émise par l'accepteur à sa longueur d'onde d'émission, puis en corrigeant le signal obtenu pour l'accepteur par celui obtenu pour le donneur, par exemple en calculant le ratio des deux valeurs. Dans ce cas, une « augmentation du FRET » correspondra à une augmentation du ratio accepteur/donneur. Les fluorimètres incluent généralement un mode permettant ce type de lecture. Par ailleurs, la luminescence des composés donneurs et accepteurs n'est pas obligatoirement mesurée dans la même fenêtre temporelle.

Il est particulièrement avantageux de procéder aux mesures de FRET en temps résolu (TR-FRET), ce qui est possible avec des composés donneurs d'énergie à durée de vie longue, puisque ce type de mesure permet d'éliminer un grand nombre d'interférences. Même si le procédé selon l'invention peut être mis en oeuvre en mode FRET , le mode TR-FRET est largement préféré.

## EXEMPLES

Les produits suivants sont utilisés dans les exemples 1 à 12 :

### *Réactifs :*

*Modulateurs (agonistes, antagonistes, modulateurs allostériques) :*

DCG IV, Acide L-Glutamique et LY341495, sont disponibles dans le catalogue Tocris Biosciences. Le 4-MPPTS (modulateur allostérique positif du récepteur mGluR2, également appelé LY487379) a été donné par Addex Pharmaceuticals, sa synthèse est décrite dans le brevet américain US 6,800,651.

*Milieux de culture :*

DMEM, DMEM+Glutamax, MEM-NEAA, MEM-Penicilline/Streptomycine, Trypsine/EDTA sont disponibles dans le catalogue Gibco. Le sérum de veau foetal utilisé est de marque Lonza, de type Sud Américain (lot 7SB0017)

*Conjugués fluorescents :*

BG-Lumi4-Tb : conjugué benzylguanine - Lumi4-Tb, commercialisé par Cisbio Bioassays (Tag-lite® SNAP-Lumi4Tb). BG est le substrat de l'enzyme Snaptag.

BC-fluorescéine : conjugué benzylcytosine - fluorescéine, commercialisé par la société NEB sous le nom commercial CLIP-Vista Green. BC est le substrat de l'enzyme Cliptag.

BG-fluorescéine : conjugué benzylguanine - fluorescéine, commercialisé par la société NEB sous le nom commercial SNAP-Vista Green (New England Biolabs).

Tag-lite® SNAP-Green : conjugué benzylguanine - fluorescéine, commercialisé par la société Cisbio Bioassays.

Tag-lite® SNAP-red : conjugué benzylguanine-dérivé de cyanine dont le pic d'émission se situe à 670nm, commercialisé par Cisbio Bioassay.

*Autres réactifs :*

DMSO, Tris (Trizma Base), NaCl, $KH_2PO_4$, $MgSO_4$, KCl, $CaCl_2$ sont disponibles dans le catalogue Sigma-Aldrich.

***<u>Plasmides utilisés pour l'expression des dimères RCPG1-RCPG2 :</u>***

Les vecteurs d'expression comportant l'ADN codant pour les protéines de fusion HA-Snaptag-[RCPG1] et Flag-Cliptag-[RCPG2], dans lesquelles RCPG1 et RCPG2 représentent les RCPGs que l'on souhaite faire exprimer par la cellule, ont été préparés à partir du vecteur prK5 par les techniques classiques de biologie moléculaire et leurs séquences ont été vérifiées par séquençage. Sa séquence qui comporte 4661 paires de base est la séquence SEQ ID N°4. Les positions des sites de restriction uniques de ce plasmide sont indiquées ci-après :

| | |
|---|---|
| SpeI | 28 |
| NdeI | 263 |
| SacII | 684 |
| EagI | 684 |
| ClaI | 913 |
| EcoRI | 919 |
| SmaI | 927 |
| BamHI | 930 |
| XbaI | 936 |
| SalI | 942 |
| PstI | 952 |
| HindIII | 954 |
| KpnI | 1173 |
| StuI | 1518 |
| HpaI | 1542 |
| NarI | 1710 |

Les vecteurs, dénommés ci-après prK5-HA-Snaptag-[RCPG1] ou pRK5-Flag-Cliptag-[RCPG2] contiennent un promoteur CMV, un codon initiateur ATG, la séquence d'ADN codant pour le peptide signal de mGluR5, un épitope de l'hémaglutinine de l'influenza (HA) ou l'épitope FLAG, la séquence d'ADN codant pour l'enzyme Snaptag ou celle de l'enzyme Cliptag, la séquence d'ADN codant pour le récepteur [RCPG1] ou [RCPG2] à l'exception de leurs codons initiateurs et des séquences codant pour leur peptide signal, s'il existe, et enfin un codon stop.

Les séquences des plasmides codant pour les protéines de fusion HA-Snaptag- $mGluR_2$ et Flag-Cliptag-$mGluR_2$ utilisés dans l'exemple 1 sont les séquences SEQ ID N°5 et SEQ ID N°6.

Dans la séquence SEQ ID N°5, la position des parties codantes est respectivement :

- peptide signal de $mGluR_5$ : 937-1002 ;
- épitope HA : 1009-1035 ;
- Snaptag : 1042-1590 ;
- $mGluR_2$ : 1597-4149.

Dans la séquence SEQ ID N°6, la position des parties codantes est respectivement :

- peptide signal de $mGluR_5$ : 937-1002 ;
- FLAG : 1015-1038 ;
- Cliptag : 1045-1593 ;
- $mGluR_2$ : 1600-4152.

Les plasmides comportant les séquences d'autres récepteurs sont identiques à l'exception de la partie codant pour le récepteur d'intérêt. Les séquences des différents récepteurs utilisés sont accessibles dans la banque de donnée Genbank.

L'homme du métier pourra facilement construire ces plasmides soit par sous-clonage à partir de plasmides déjà décrits dans la littérature (Maurel & al. cité précédemment), soit directement par synthèse du gène.

***Cellules* :** On a utilisé la lignée cellulaire Cos-7 qui est disponible commercialement.

***Milieu complet* :** DMEM + 10% sérum de veau foetal + MEM-NEAA + MEM - Pénicilline/Streptomycine.

***Tampon d'électroporation* :** $KH_2PO_4$ 50mM, $CH_3COOK$ 20mM, KOH 20mM, $MgSO_4$ 50mM.

***Tampon de marquage* :** DMEM+Glutamax.

***Tampon de lavage et de lecture* :** Tris 20mM, NaCl 118mM, $KH_2PO_4$ 1,2mM, $MgSO_4$ 1,2mM, KCl 4,7mM, $CaCl_2$ 1,8mM ; ajustement du pH à 7,4.

**EXEMPLE 1 : Mise en évidence d'un composé activateur à effet agoniste sur un dimère $mGluR_2$-$mGluR_2$** ($R_0$ du couple de partenaires de FRET utilisé : 48 Å) **Protocole :**

***Transfection par électroporation :***

Les cellules Cos-7 ont été cultivées dans du milieu complet à 37°C et 5% de $CO_2$. A confluence, elles ont été lavées une fois avec du PBS, et décollées avec une solution de Trypsine-EDTA pendant 10min à 37°C. Une suspension de 10 millions de cellules a été mélangée à 5 μg d'ADN total (3,5 μg de plasmide prK5, 1,2 μg de plasmide prK5 Flag-Cliptag-mGluR2 et 0,3 μg de plasmide prK5 HA-Snaptag-mGluR2), dans un volume final de 300μl de tampon d'électroporation, puis a été soumise à un choc électrique de 280V, 900μF à l'aide d'un électroporateur Gene-Pulser II (BIO-RAD), dans des cuvettes d'électroporation 4mm (Eurogentec). Les cellules ont été ensuite ensemencées dans une plaque 96 puits (Greiner CellStar 96-well plate), à 150 000 cellules par puits, dans un volume final de 100μl par puits de milieu complet, puis ont été incubées 24 heures.

***Marquage***

24 heures après la transfection, les cellules ont été incubées 2h à 37°C en présence de 50μl de tampon de marquage + 0,3μM BG-Lumi4-Tb et 1μM BC-fluorescéine.

Les cellules ont ensuite été lavées 4 fois avec 100μl de tampon de lavage, et la lecture a été effectuée dans 100μl du tampon de lavage.

***Lecture***

La fluorescence émise à la longueur d'onde de la fluoréscéine (accepteur) a été lue sur un lecteur de microplaques Analyst AD (Molecular Devices) en mode TRF, délai 50μs, temps d'intégration 450μs, filtre d'excitation 320nm bande passante (BP) 25, miroir dichroïque BB/UV, filtres d'émission 520nm BP10. Ce signal de FRET est ci-après désigné TRF520.

La plaque a été lue une première fois en l'absence de glutamate.

20μl de solution de glutamate a ensuite été ajoutée aux 100μl de tampon de lecture, de façon à obtenir les concentrations finales dans le puits (volume total de 120μl) de 316μM ; 100mM ; 31,6μM ; 10μM ; 3,16M ; 1μM ; ou 0,316μM.

Après l'ajout du glutamate, la plaque a été lue une deuxième fois.

***Résultats***

La figure 1 représente le signal TRF520 en fonction de la concentration en glutamate. Ce signal est exprimé en pourcentage du TRF520 lu à la première lecture c'est-à-dire avant ajout du glutamate, c'est-à-dire :

$$\text{TRF520 (\%)} = \frac{\text{signal TRF520 lu après ajout du glutamate}}{\text{signal TRF520 lu avant ajout du glutamate}} \times 100$$

En présence d'agoniste (glutamate), une diminution du TRF520 est observée, et n'est attribuable ni à une diminution de la fluorescence du cryptate, ni à une diminution de la fluorescence de l'accepteur. Cela indique qu'il y a eu une variation de l'efficacité de transfert d'énergie (FRET) entre les composés donneur et accepteur, pouvant notamment être due à une variation de distance entre les fluorophores.

Cette variation est dose-dépendante, c'est-à-dire qu'elle dépend de la concentration de l'agoniste glutamate. La forme de la courbe est une sigmoïde ; la concentration nécessaire pour avoir une variation de TRF520 égale à la moitié de la variation maximale correspond étroitement à l'$EC_{50}$ pharmacologique du glutamate sur le récepteur mGluR2. Cela suggère fortement que le phénomène observé lors de la variation de TRF520 est l'activation du récepteur.

Cet exemple montre que le procédé selon l'invention permet de mettre en évidence des composés activateurs du dimère, en particulier des composés agonistes. Dans le cas de l'homodimère $mGluR_2$, les composés activateurs provoquent une baisse du signal TRF520.

**<u>EXEMPLE 2 :</u> Mise en évidence d'un composé désactivateur à effet antagoniste compétitif sur un dimère mGluR2-mGluR2** ($R_0$ du couple de partenaires de FRET utilisé : 48 Å)

***Réactifs et plasmides***

On a utilisé les mêmes réactifs et plasmides que ceux décrits à l'exemple 1. Le composé antagoniste connu, LY341495 (Tocris Bioscience) a été utilisé.

***Protocole***

On a utilisé le même protocole que celui décrit à l'Exemple 1. Les concentrations finales étaient [Glutamate] = 100μM dans tous les puits et [LY341495] = 1000nM ; 316nM ; 100nM ; 31,6nM ; 10nM ; 3,16nM ; ou 1nM.

***Résultats***

La figure 2 représente le signal TRF520 en fonction de la concentration en antagoniste LY341495 et en présence d'une concentration constante de glutamate (courbe contrôle : en l'absence de glutamate).

En présence de glutamate et en l'absence de l'antagoniste compétitif LY341495, le récepteur est activé, et on observe une variation de TRF520 de 50%. La présence d'antagoniste empêche cette action du glutamate sur le TRF520 et le signal mesuré en présence de concentrations croissantes d'antagoniste augmente, à l'inverse de ce qui est observé en présence de concentrations croissantes d'agoniste (exemple 1). Lorsque la concentration en antagoniste est maximale (1μM), la variation de FRET induite par l'agoniste est entièrement inhibée (100%). La concentration nécessaire pour inhiber 50% de la variation de FRET induite par le glutamate, ici 150 nM, est peu différente de la concentration nécessaire pour inhiber 50% de l'action pharmacologique du glutamate ($IC_{50}$).

Cet exemple montre l'efficacité du procédé selon l'invention pour mettre en évidence l'effet désactivateur d'un composé antagoniste connu. En procédant de manière similaire avec un composé à tester, on peut déterminer si ce composé à un effet antagoniste.

**<u>EXEMPLE 3</u> : Mise en évidence d'un composé activateur à effet agoniste partiel sur un dimère mGluR2-mGluR2** ($R_0$ du couple de partenaires de FRET utilisé : 48 Å)

***Réactifs et plasmides***

On a utilisé les mêmes réactifs et plasmides que ceux décrits à l'exemple 1. Le composé agoniste partiel connu utilisé était le DCG-IV (Tocris Bioscience) ; il a été comparé à l'agoniste complet de référence, le glutamate.

*Protocole*

Le protocole de l'exemple 1 a été utilisé. Les concentrations finales en [DCG-IV] étaient les suivantes : 100μM ; 31,6μM ; 10μM ; 3,16μM ; 1μM ; 0,316μM ; ou 0,1μM.

*Résultats*

La figure 3 représente le signal TRF520 en fonction de la concentration en Glutamate (agoniste complet) ou en DCG-IV (agoniste partiel).

De même que le glutamate, le DCG-IV est capable d'inhiber le signal TRF520 de façon dose-dépendante. La concentration nécessaire pour obtenir une variation égale à la moitié de la variation maximale est proche de l'$EC_{50}$ pharmacologique du DCG-IV (0,4μM). Néanmoins, la variation maximale obtenue avec le DCG-IV est de 30% alors qu'elle est de 50% avec le glutamate. Ce résultat confirme le caractère agoniste partiel du DCG-IV qui provoque une variation maximale de FRET inférieure à celle obtenue avec l'agoniste de référence.

Cet exemple montre que le procédé selon l'invention peut être utilisé pour sélectionner des composés ayant un effet activateur de type agoniste partiel sur les dimères de $mGluR_2$.

**<u>EXEMPLE 4 :</u> Mise en évidence d'un composé activateur à effet modulateur allostérique positif (PAM) sur un dimère mGluR2-mGluR2** ($R_0$ du couple de partenaires de FRET utilisé : 48 Å)

*Réactifs et plasmides*

Les réactifs et plasmides de l'Exemple 1 ont été utilisés. Un composé modulateur allostérique positif connu a été utilisé, le 4-MPPTS (= le PAM $mGluR_2$, aussi appelé LY487379).

*Protocole*

On a utilisé le protocole décrit à l'exemple 1.

Les concentrations finales étaient [4-MPPTS] = 10μM et [Glutamate] = 1000μM ; 316μM ; 100μM ; 31,6μM ; 10μM ; 3,16μM ; ou 1μM.

*Résultats*

La figure 4 représente l'évolution du signal TRF520 en fonction de la concentration de glutamate en présence ou en l'absence (contrôle) de 4-MPPTS.

Pour une concentration donnée en glutamate, on observe une diminution du signal plus importante en présence de 4-MPPTS qu'en son absence.

Cet exemple montre que le procédé selon l'invention peut être utilisé pour mettre en évidence des composés ayant un effet modulateur allostérique positif.

**<u>EXEMPLE 5 :</u> Un marquage avec deux enzymes suicides différentes n'est pas requis** ($R_0$ du couple de partenaires de FRET utilisé : 48 Å)

Dans cet exemple un seul plasmide a été utilisé, le prK5-HA-Snaptag-$mGluR_2$, pour exprimer les homodimères $mGluR_2$. Le protocole est le même que celui utilisé dans l'exemple 3, à ceci près que les quantités d'ADN transfecté sont : 4 μg de prK5 + 1ug de pRK5-HA-Snaptag-mGluR2.

On a utilisé les conjugués fluorescents ci-après : BG-Lumi4-Tb à une concentration de 0,1μM dans le milieu de marquage et le BG-fluorescéine à 0,02μM.

La figure 5 montre l'évolution du signal de FRET en fonction de concentrations croissantes de glutamate et de DGC-IV et montre que chacune des protéines formant le dimère, ici chacun des mGluR2 formant l'homodimère, peut être marquée de la même manière, ici par une approche BG/Snaptag. L'utilisation de deux enzymes suicides différentes n'est donc pas indispensable.

**<u>EXEMPLE 6 :</u> Mise en évidence d'un composé activateur à effet agoniste sur les homodimères mGluR2-mGluR2 mGluR3-mGluR3 ou mGluR4-mGluR4** ($R_0$ du couple de partenaires de FRET utilisé : 48 Å)

L'exemple 1 a été reproduit avec des plasmides permettant l'expression soit d'homodimères mGluR3-mGluR3 soit d'homodimères mGluR4-mGluR4. La procédure pour obtenir les plasmides prK5-HA-Snaptag-mGluR3, prK5-Flag-Cliptag-mGluR3, prK5-HA-Snaptag-mGluR4 et prK5-Flag-Cliptag-mGluR4 est décrite dans la partie « plasmides utilisés ». Le protocole décrit dans l'exemple 1 a été suivi, à ceci près que les quantités d'ADN transfecté sont :

- pour mGluR2 : identique à l'exemple 1 ;
- pour mGluR3 : 3,5 µg de plasmide prK5, 1,2 µg de plasmide prK5 Flag-Cliptag-mGluR3 et 0,3 µg de plasmide prK5 HA-Snaptag-mGluR3 ;
- pour mGluR4 : 3,5 µg de plasmide prK5, 1,2 µg de plasmide prK5 Flag-Cliptag-mGluR4 et 0,3 µg de plasmide prK5 HA-Snaptag-mGluR4.

Les courbes dose-réponse obtenues sont représentées sur la figure 6. Les résultats obtenus dans l'exemple 1 avec l'homodimère mGluR2-mGluR2 sont confirmés avec mGluR4-mGluR4 et mGluR3-mGluR3, même si la variation de signal observée sur ce dernier récepteur est moins importante que celle mesurée dans les autres cas.

Cet exemple montre que le procédé selon l'invention peut être mis en oeuvre avec les différents récepteurs métabotropiques au glutamate.

**EXEMPLE 7 : Le cas du senseur mGluR3: sensible à l'antagoniste, peu sensible à l'agoniste** ($R_0$ du couple de partenaires de FRET utilisé : 48 Å)

Dans cet exemple, le protocole de l'exemple 1 a été utilisé avec les plasmides et les quantités ci-après :

- 3,5 µg de plasmide prK5, 1,2 µg de plasmide prK5 Flag-Cliptag-mGluR3 et 0,3 µg de plasmide prK5-HA-Snaptag-mGluR3 ont été utilisés, pour obtenir l'expression de l'homodimère mGluR3 marqué.
- Des concentrations croissantes de l'antagoniste LY 341495 ont été utilisées pour obtenir les concentrations finales : 1 µM, 320 nM, 100 nM, 32 nM, 10 nM, 3,2 nM et 1 nM.

***Résultats***

La figure 7 représente le signal TRF520 en fonction de la concentration en LY341495.

L'addition de l'antagoniste provoque une augmentation du signal TRF520, de façon dose-dépendante.

Cet exemple montre que le procédé selon l'invention peut être mis en oeuvre pour cribler des molécules désactivatrices du récepteur d'intérêt.

**EXEMPLE 8 : Mise en évidence d'un composé activateur à effet agoniste sur les homodimères mGluR2-mGluR2, mGluR3-mGluR3 ou mGluR4-mGluR4 et les hétérodimères mGluR2-mGluR3 et mGluR2-mGluR4** ($R_0$ du couple de partenaires de FRET utilisé : 48 Å)

L'exemple 1 a été reproduit avec les plasmides permettant l'expression d'homodimères mGluR2-mGluR2, mGluR3-mGluR3, mGluR4-mGluR4 ou d'hétérodimères mGluR2-mGluR3 et mGluR2-mGluR4. Les courbes dose-réponse obtenues sont représentées sur la figure 8. Les résultats obtenus dans les exemples 1 et 6 sont confirmés.

Cet exemple montre que le procédé selon l'invention peut être mis en oeuvre non seulement avec des homodimères mais également avec des hétérodimères inter sous-types de récepteurs métabotropiques au glutamate.

**EXEMPLE 9 : Mise en évidence de l'effet d'un composé modulateur sur les homodimères mGluR1-mGluR1 et mGluR8-mGluR8** ($R_0$ du couple de partenaires de FRET utilisé : 48 Å)

Des cellules exprimant les homodimères mGluR1-mGluR1, mGluR2-mGluR2, mGluR3-mGluR3 et mGluR8-mGluR8 ont été préparées selon une approche similaire à celle décrite dans l'exemple 5, chaque récepteur mGluR étant exprimé sous forme de protéine de fusion avec l'enzyme Snaptag.

Le marquage a été effectué avec les conjugués fluorescents ci-après : BG-Lumi4-Tb (donneur) à une concentration de 0,1µM dans le milieu de marquage et le BG-fluorescéine (accepteur) à 0,1µM.

La fluorescence émise à la longueur d'onde de la fluorescéine (520nm) et du Terbium (620nm) ont été mesurées sur un lecteur de microplaques Infinit 500 avec un délai de 50µs, et un temps d'intégration de 400µs.

Ces mesures ont été effectuées en présence de tampon de marquage, ou bien d'agoniste (1 mM glutamate) ou d'antagoniste (0,1 mM LY341495).

Le ratio des signaux émis à 520nm et 620nm a été calculé, et est dénommé ci-après « ratio de FRET ».

La figure 9 représente la variation de ratio de FRET observée en présence d'agoniste (glutamate), d'antagoniste (LY341495), ou en l'absence de ces composés (tampon). La valeur 100% est choisie arbitrairement comme celle observée en présence d'antagoniste.

Ces résultats confirment ceux obtenus dans les exemples précédents pour les homodimères de mGluR2 et de mGluR3, et montrent également que la méthode selon l'invention permet d'observer une variation du signal de FRET lorsque les homodimères de mGluR8 et mGluR1 sont mis en présence d'agonistes ou d'antagonistes.

<u>**EXEMPLE 10**</u> **: Mise en évidence de l'effet d'un composé modulateur sur les homodimères de mGluR1, mGluR2, mGluR3, mGluR4, mGluR5 et mGluR8** ($R_0$ du couple de partenaires de FRET utilisé : 48 Å)

L'exemple 9 a été reproduit avec les homodimères de mGluR1, mGluR2, mGluR3, mGluR4, mGluR5 et mGluR8, et la fluorescence a cette fois été mesurée avec un délai de 500 μs et un temps d'intégration de 1000 μs.

Les résultats de la figure 10 confirment ceux observés précédemment, à savoir que le procédé selon l'invention est adapté à la mise en évidence de composés modulateurs de la plupart des récepteurs mGluR.

**EXEMPLE 11 : Mise en évidence de l'effet d'un composé modulateur sur les homodimères de mGluR8** ($R_0$ du couple de partenaires de FRET utilisé : 58 Å)

L'exemple 9 a été reproduit avec une cellule exprimant le dimère mGluR8-mGluR8, mais les composés fluorescents utilisés ont un R0>55Å. En l'occurrence, le marquage a été effectué avec BG-Lumi4Tb (donneur, 0,1 μM) et Tag-lite® SNAP- Red (accepteur, 0,25 μM).

La fluorescence a été mesurée sur un lecteur Rubystar à 665nm (accepteur) et 620nm (donneur) dans les fenêtres temporelles suivantes : 50-450μs, 50-150μs et 500-1500 μs (figure 11).

On n'observe pas de variation significative de signal en présence de composé modulateur (agoniste où antagoniste) lorsque le signal est mesuré dans la fenêtre classiquement utilisée, à savoir 50-450μs. Une variation significative en présence d'agoniste est observée lorsque la fluorescence est mesurée dans la fenêtre temporelle 50-150 μs. Des variations très significatives en présence d'agoniste ou d'antagoniste sont observées lorsque la fluorescence est mesurée dans la fenêtre temporelle 500-1500 μs.

**EXEMPLE 12 : Mise en évidence de l'effet d'un composé modulateur sur les homodimères de mGluR8 et mGluR2 avec un couple de partenaires de FRET dont le RO>55 Å : BG-TrisBiPy-pentaacide-Eu et Tag-lite® SNAP-Red** ($R_0$ du couple de partenaires de FRET utilisé : 58 Å)

L'exemple 9 a été reproduit avec des cellules exprimant le dimère mGluR8-mGluR8 ou le dimère mGluR2-mGluR2, mais les composés fluorescents utilisés ont un R0>55Å. En l'occurrence, le marquage a été effectué avec un cryptate d'Europium couplé à la benzylguanine (**BG-TrisBiPy-pentaacide-Eu,** donneur, 0,2 μM) et Tag-lite® SNAP-Red (accepteur, 0,25 μM).

La fluorescence a été mesurée sur un lecteur Rubystar à 665 (accepteur) et 620 (donneur) dans les fenêtres temporelles suivantes : 50-450μs et 300-650 μs (figure 12).

On n'observe pas de variation significative de signal en présence de composé modulateur (agoniste ou antagoniste) lorsque le signal est mesuré dans la fenêtre classiquement utilisée, à savoir 50-450μs. Des variations significatives en présence d'agoniste ou d'antagoniste sont observées lorsque la fluorescence est mesurée dans la fenêtre temporelle 300-650 μs.

## Revendications

1. Procédé de sélection de composés ayant un effet modulateur sur l'état d'activation d'un dimère de protéines à domaine VFT exprimées dans des membranes cellulaires présentes dans un milieu de mesure, ledit dimère étant constitué d'une première protéine et d'une seconde protéine, lesdites protéines étant identiques ou différentes, procédé qui comprend les étapes suivantes :

   (a) marquage de la première et de la seconde protéine dans la partie N-terminale de leurs domaines VFT par les membres d'un couple de partenaires de FRET;
   (b) excitation des partenaires de FRET et mesure du signal de FRET en l'absence et en présence du composé à tester ;
   (c) sélection du composé à tester comme composé modulateur si une différence de signal de FRET en l'absence et en présence de composé à tester est mesurée à l'étape (b),

   le signal de FRET étant mesuré à l'étape (b) dans une fenêtre temporelle dans laquelle les signaux mesurés en présence et en l'absence d'un composé agoniste ou antagoniste de référence, sont différents.

2. Procédé selon la revendication 1, **caractérisé en ce que** le signal mesuré à l'étape (b) l'est avec un délai compris entre 180 et 800 μs après l'excitation et un temps d'intégration de 200 à 1000 μs.

**3.** Procédé selon la revendication 1, **caractérisé en ce que** :

(i) soit le rayon de Förster ($R_0$) dudit couple de partenaires de FRET est compris entre 18 et 55 Å, et le signal mesuré à l'étape (b) l'est avec un délai compris entre 20 et 100 µs après l'excitation et un temps d'intégration de 100 à 500 µs ; ou
(ii) soit le rayon de Förster ($R_0$) dudit couple est supérieur à 55 Å, et le signal mesuré à l'étape (b) l'est avec un délai compris entre 180 et 800 µs après l'excitation et un temps d'intégration de 200 à 1000 µs.

**4.** Procédé selon l'une quelconque des revendications 1 à 3, **caractérisé en ce que** les mesures de FRET en présence et l'absence de composé à tester sont effectuées en présence d'un agoniste connu dudit dimère.

**5.** Procédé selon l'une quelconque des revendications 1 à 4, **caractérisé en ce que** lesdites première et seconde protéines sont des récepteurs couplés aux protéines G de classe C à domaine VFT.

**6.** Procédé selon l'une quelconque des revendications 1 à 5, **caractérisé en ce que** ledit dimère de protéines à domaine VFT est choisi parmi : un récepteur métabotropique du glutamate, le récepteur de l'acide gamma-aminobutyrique, le récepteur lié à la perception du goût sucré, le récepteur lié à la perception du goût umami, le récepteur sensible au calcium extracellulaire, le récepteur des acides aminés basiques.

**7.** Procédé selon l'une quelconque des revendications 1 à 5, **caractérisé en ce que** le dimère de protéines à domaine VFT est un récepteur métabotropique du glutamate, ou est choisi parmi TAS1R2-TAS1R3 ou TAS1R1-TAS1R3.

**8.** Procédé selon l'une quelconque des revendications 1 à 7, **caractérisé en ce que** le marquage de la première et de la seconde protéine par les membres d'un couple de partenaire de FRET est soit un marquage indirect par l'intermédiaire d'un couple de partenaires de liaison, soit un marquage direct par liaison covalente.

**9.** Procédé selon la revendication 8, **caractérisé en ce que** lesdites première et seconde protéines sont chacune exprimées sous forme d'une protéine de fusion avec une enzyme suicide, et **en ce que** les marquages de la première et de la seconde protéine sont effectués par addition dans le milieu de mesure des membres dudit couple de partenaires de FRET, l'un et l'autre étant chacun lié de manière covalente au substrat de ladite enzyme suicide.

**10.** Procédé selon la revendication 9, **caractérisé en ce que** la première et la seconde protéine sont chacune exprimées sous forme d'une protéine de fusion avec une enzyme suicide différente.

**11.** Procédé selon la revendication 9, **caractérisé en ce que** la première et la seconde protéine sont chacune exprimées sous forme d'une protéine de fusion avec la même enzyme suicide.

**12.** Procédé selon l'une quelconque des revendications 9 à 11, **caractérisé en ce que** la ou les enzymes suicides sont choisies parmi : les mutants de l'O6-alkylguanine DNA alkyltransférase, les mutants de la déshalogénase, ou un fragment de la protéine de transport d'acyles.

**13.** Procédé selon l'une quelconque des revendications 1 à 12, **caractérisé en ce que** ledit couple de partenaires de FRET est constitué d'une part :

(i) d'un composé donneur fluorescent choisi parmi : un chélate d'europium, un chélate de terbium, un cryptate d'europium, un cryptate de terbium et, d'autre part,
(ii) d'un composé fluorescent accepteur qui est une molécule organique fluorescente choisie parmi : les rhodamines, les cyanines, les squaraines, les coumarines, les proflavines, les acridines, les fluorescéines, le nitrobenzoxadiazole, les protéines fluorescentes cyans, les protéines fluorescentes vertes, les protéines fluorescentes jaunes, les protéines fluorescentes oranges et rouges, les protéines fluorescentes dans le rouge lointain.

**Patentansprüche**

**1.** Verfahren zur Auswahl von Verbindungen, die einen modulierenden Effekt auf den Aktivierungszustand eines Dimers von VFT-Domäne-Proteinen, welche in in einem Messmedium vorhandenen Zellmembranen exprimiert sind, haben, wobei das Dimer von einem ersten Protein und von einem zweiten Protein gebildet ist, wobei die Proteine identisch oder unterschiedlich sind, Verfahren, das die folgenden Schritte umfasst:

(a) Markieren des ersten und des zweiten Proteins in dem N-terminalen Teil ihrer VFT-Domänen mit den Mitgliedern eines Paares von FRET-Partnern,

(b) Anregen der FRET-Partner und Messen des FRET-Signals bei Abwesenheit und in Gegenwart der zu testenden Verbindung,

(c) Auswählen der zu testenden Verbindung als modulierende Verbindung, wenn bei Schritt (b) eine FRET-Signal-Differenz bei Abwesenheit und in Gegenwart der zu testenden Verbindung gemessen wird,

wobei das FRET-Signal bei Schritt (b) in einem Zeitfenster gemessen wird, in dem die Signale, die in Gegenwart und bei Abwesenheit einer Referenz-Agonisten- oder Antagonisten-Verbindung gemessen werden, unterschiedlich sind.

**2.** Verfahren nach Anspruch 1, **dadurch gekennzeichnet, dass** das bei Schritt (b) gemessene Signal mit einer Verzögerung von 180 bis 800 µs nach der Anregung und einer Integrationszeit von 200 bis 1000 µs gemessen wird.

**3.** Verfahren nach Anspruch 1, **dadurch gekennzeichnet, dass**:

(i) entweder der Förster-Radius ($R_0$) des Paares von FRET-Partnern im Bereich zwischen 18 und 55 Å liegt und das bei Schritt (b) gemessene Signal mit einer Verzögerung von 20 bis 100 µs nach der Anregung und einer Integrationszeit von 100 bis 500 µs gemessen wird, oder

(ii) der Förster-Radius ($R_0$) des Paares mehr als 55 Å beträgt und das bei Schritt (b) gemessene Signal mit einer Verzögerung von 180 bis 800 µs nach der Anregung und einer Integrationszeit von 200 bis 1000 µs gemessen wird.

**4.** Verfahren nach einem der Ansprüche 1 bis 3, **dadurch gekennzeichnet, dass** die FRET-Messungen in Gegenwart und bei Abwesenheit der zu testenden Verbindung in Gegenwart eines bekannten Agonisten des Dimers durchgeführt werden.

**5.** Verfahren nach einem der Ansprüche 1 bis 4, **dadurch gekennzeichnet, dass** das erste und das zweite Protein G-Protein-gekoppelte Rezeptoren der Klasse C mit VFT-Domäne sind.

**6.** Verfahren nach einem der Ansprüche 1 bis 5, **dadurch gekennzeichnet, dass** das Dimer von VFT-Domäne-Proteinen ausgewählt ist aus: einem metabotropen Glutamatrezeptor, dem Rezeptor der gamma-Aminobuttersäure, dem mit der Wahrnehmung des Süßgeschmacks verbundenen Rezeptor, dem mit der Wahrnehmung des Umami-Geschmacks verbundenen Rezeptor, dem gegenüber extrazellulärem Calcium empfindlichen Rezeptor, dem Rezeptor der basischen Aminosäuren.

**7.** Verfahren nach einem der Ansprüche 1 bis 5, **dadurch gekennzeichnet, dass** das Dimer von VFT-Domäne-Proteinen ein metabotroper Glutamatrezeptor ist oder aus TAS1 R2-TAS1 R3 oder TAS1R1-TAS1R3 ausgewählt ist.

**8.** Verfahren nach einem der Ansprüche 1 bis 7, **dadurch gekennzeichnet, dass** die Markierung des ersten und des zweiten Proteins mit den Mitgliedern eines Paares von FRET-Partnern entweder eine indirekte Markierung mittels eines Paares von Bindungspartnern oder eine direkte Markierung durch kovalente Bindung ist.

**9.** Verfahren nach Anspruch 8, **dadurch gekennzeichnet, dass** das erste und das zweite Protein jeweils in Form eines Fusionsproteins mit einem Suizid-Enzym exprimiert sind und dass die Markierungen des ersten und des zweiten Proteins durch Zugabe der Mitglieder des Paares von FRET-Partnern in das Messmedium vollzogen werden, wobei das eine und das andere jeweils kovalent an das Substrat des Suizid-Enzyms gebunden sind.

**10.** Verfahren nach Anspruch 9, **dadurch gekennzeichnet, dass** das erste und das zweite Protein jeweils in Form eines Fusionsproteins mit einem unterschiedlichen Suizid-Enzym exprimiert sind.

**11.** Verfahren nach Anspruch 9, **dadurch gekennzeichnet, dass** das erste und das zweite Protein jeweils in Form eines Fusionsproteins mit dem gleichen Suizid-Enzym exprimiert sind.

**12.** Verfahren nach einem der Ansprüche 9 bis 11, **dadurch gekennzeichnet, dass** das oder die Suizid-Enzyme ausgewählt sind aus: den Mutanten der 06-Alkylguanin-DNA-Alkyltransferase, den Mutanten der Dehalogenase oder einem Fragment des Acyl-Carrier-Proteins.

**13.** Verfahren nach einem der Ansprüche 1 bis 12, **dadurch gekennzeichnet, dass** das Paar von FRET-Partnern einerseits besteht aus:

(i) einer fluoreszierenden Donator-Verbindung, die ausgewählt ist aus: einem Europiumchelat, einem Terbiumchelat, einem Europiumkryptat, einem Terbiumkryptat und, andererseits, aus
(ii) einer fluoreszierenden Akzeptor-Verbindung, die ein fluoreszierendes organisches Molekül ist, das ausgewählt ist aus: den Rhodaminen, den Cyaninen, den Squarainen, den Cumarinen, der Proflavinen, den Akridinen, den Fluoresceinen, Nitrobenzoxadiazol, den cyan-fluoreszierenden Proteinen, den grün-fluoreszierenden Proteinen, den gelb-fluoreszierenden Proteinen, den orange- und rot-fluoreszierenden Proteinen, den im fernen Rot fluoreszierenden Proteinen.

## Claims

**1.** A method for selecting compounds having a modulating effect on the activation state of a dimer of VFT-domain proteins expressed in cell membranes present in a measuring medium, said dimer consisting of a first protein and of a second protein, said proteins being identical or different, wherein the method comprises the following steps:

(a) labeling the first and second protein in the N-terminal portion of their VFT domains with the members of a pair of FRET partners;
(b) exciting the FRET partners and measuring the FRET signal in the absence and in the presence of a test compound;
(c) selecting the test compound as a modulating compound if a difference in FRET signal in the absence and in the presence of test compound is measured in step (b),

the FRET signal being measured in step (b) within a time window in which the signals measured in the presence and in the absence of a reference agonist or antagonist compound are different.

**2.** The method as claimed in claim 1, **characterized in that** the signal measured in step (b) is measured with a delay of between 180 and 800 μs after excitation and an integration time of from 200 to 1000 μs.

**3.** The method as claimed in claim 1, **characterized in that**:

(i) either the Förster radius ($R_0$) of said pair of FRET partners is between 18 and 55 Å, and the signal measured in step (b) is measured with a delay of between 20 and 100 μs after excitation and an integration time of from 100 to 500 μs;
(ii) or the Förster radius ($R_0$) of said pair is greater than 55 Å, and the signal measured in step (b) is measured with a delay of between 180 and 800 μs after excitation and an integration time of from 200 to 1000 μs.

**4.** The method as claimed in any one of claims 1 to 3, **characterized in that** the FRET measurements in the presence and the absence of test compound are carried out in the presence of a known agonist of said dimer.

**5.** The method as claimed in any one of claims 1 to 4, **characterized in that** said first and second proteins are class C G-protein coupled receptors comprising a VFT domain.

**6.** The method as claimed in any one of claims 1 to 5, **characterized in that** said dimer of VFT-domain proteins is chosen from: a metabotropic glutamate receptor, the gamma-amino butyric acid receptor, the receptor linked to sweet taste perception, the receptor linked to umami taste perception, the extracellular calcium-sensing receptor and the basic amino acid receptor.

**7.** The method as claimed in any one of claims 1 to 5, **characterized in that** the dimer of VFT-domain proteins is a metabotropic glutamate receptor, or is chosen from TAS1R2-TAS1R3 or TAS1R1-TAS1R3.

**8.** The method as claimed in any one of claims 1 to 7, **characterized in that** the labeling of the first and of the second protein with the members of a pair of FRET partners is either indirect labeling by means of a pair of binding partners, or direct labeling by covalent bonding.

**9.** The method as claimed in claim 8, **characterized in that** said first and second proteins are each expressed in the

form of a fusion protein with a suicide enzyme, and **in that** the labeling of the first and of the second protein is carried out by adding, to the measuring medium, the members of said pair of FRET partners, each one being covalently bonded to the substrate of said suicide enzyme.

**10.** The method as claimed in claim 9, **characterized in that** the first and second proteins are each expressed in the form of a fusion protein with a different suicide enzyme.

**11.** The method as claimed in claim 9, **characterized in that** the first and second proteins are each expressed in the form of a fusion protein with the same suicide enzyme.

**12.** The method as claimed in any one of claims 9 to 11, **characterized in that** the suicide enzyme(s) is (are) chosen from: O6-alkylguanine DNA alkyltransferase mutants, dehalogenase mutants, or a fragment of the acyl carrier protein.

**13.** The method as claimed in any one of claims 1 to 12, **characterized in that** said pair of FRET partners consists:

(i) on the one hand, of a fluorescent donor compound chosen from: a europium chelate, a terbium chelate, a europium cryptate and a terbium cryptate and,
(ii) on the other hand, of an acceptor fluorescent compound which is a fluorescent organic molecule chosen from: rhodamines, cyanins, squaraines, coumarins, proflavins, acridines, fluoresceins, nitrobenzoxadiazole, cyan fluorescent proteins, green fluorescent proteins, yellow fluorescent proteins, orange and red fluorescent proteins, and proteins that are fluorescent in the far-red range.

**Figure 1**

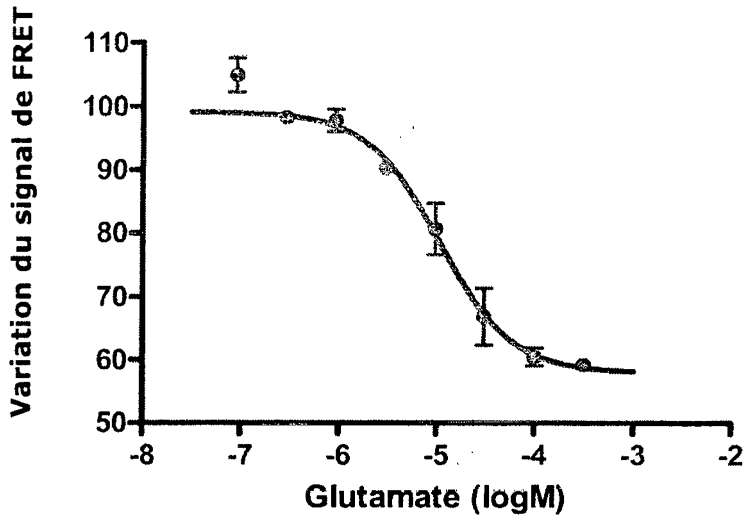


**Figure 2**

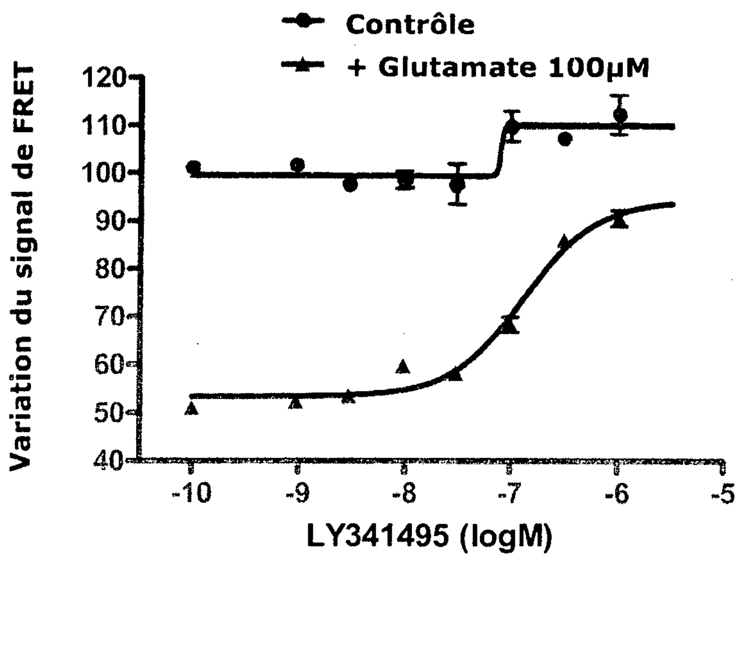

**Figure 3**

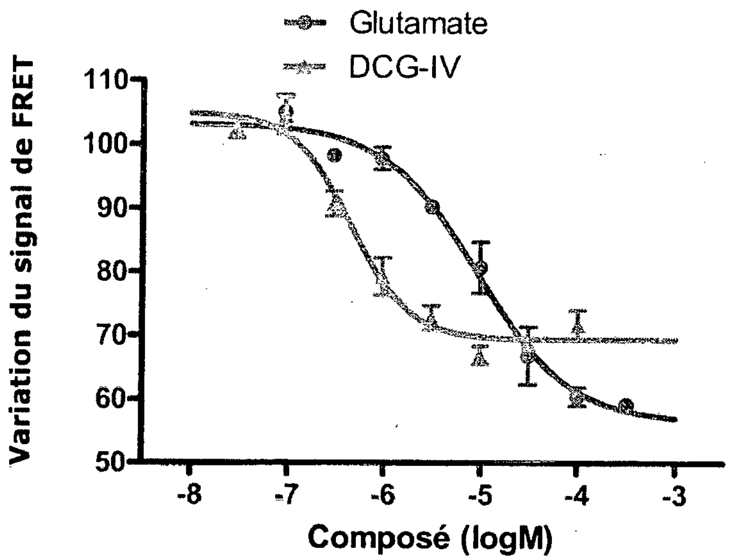


**Figure 4**

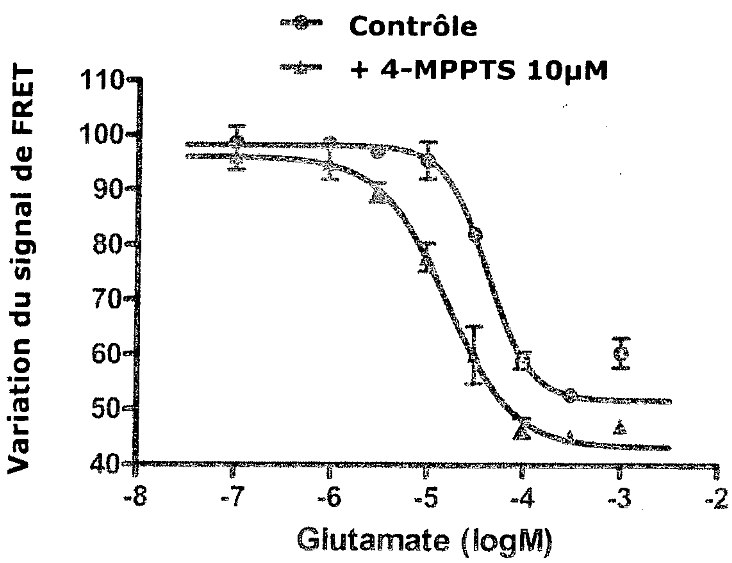

## Figure 5

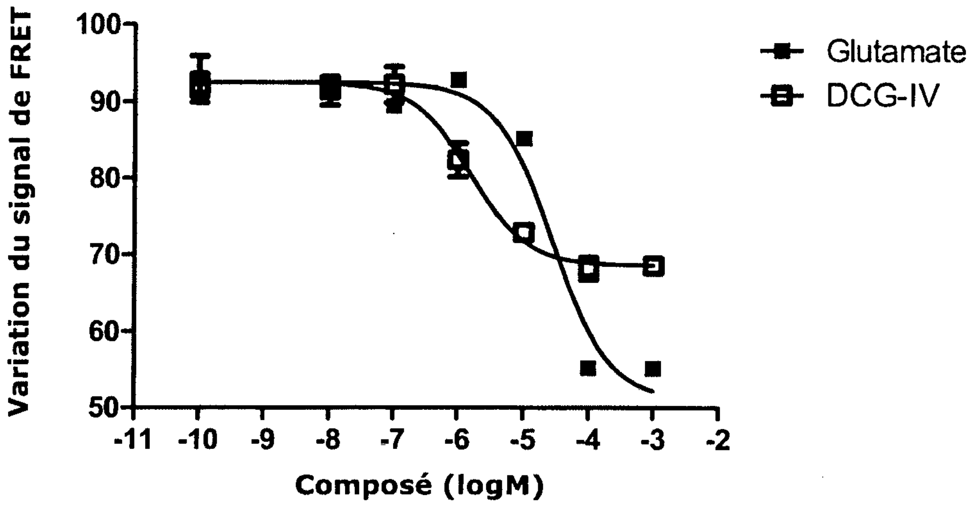


## Figure 6

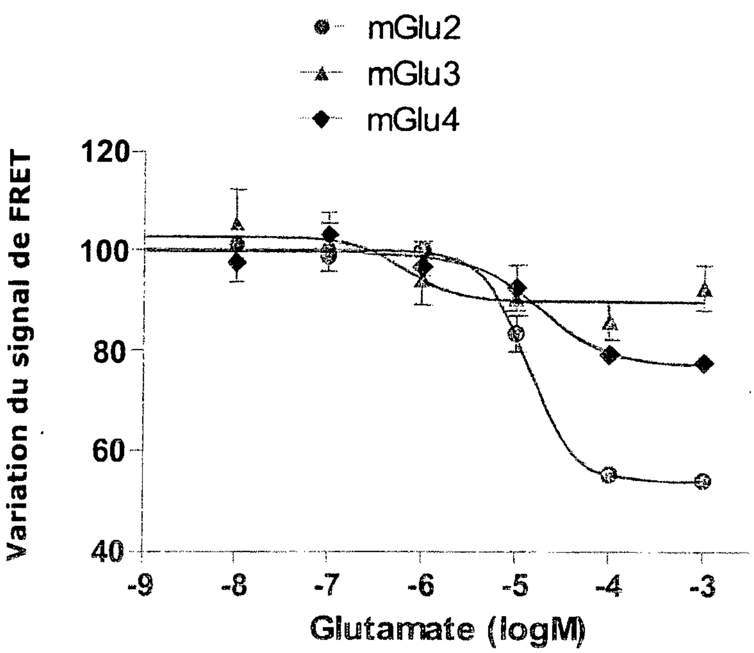

## Figure 7

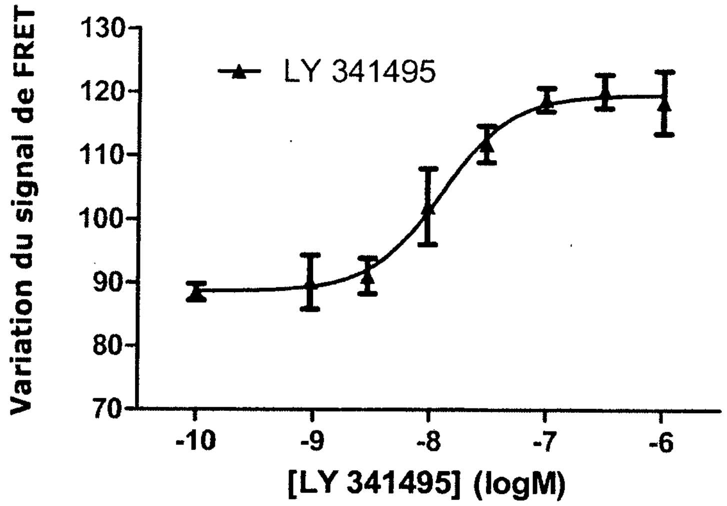


## Figure 8

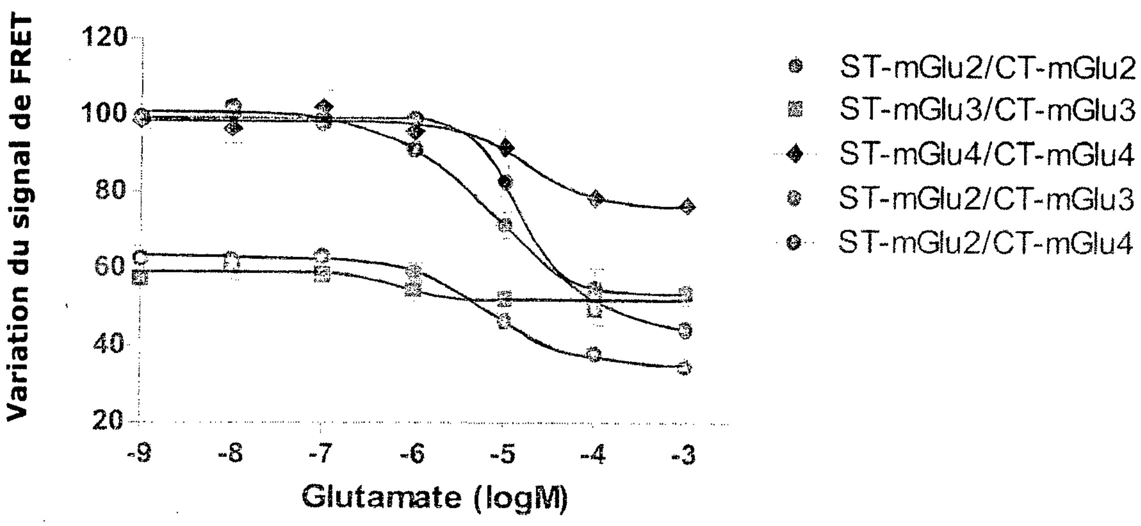

Figure 9

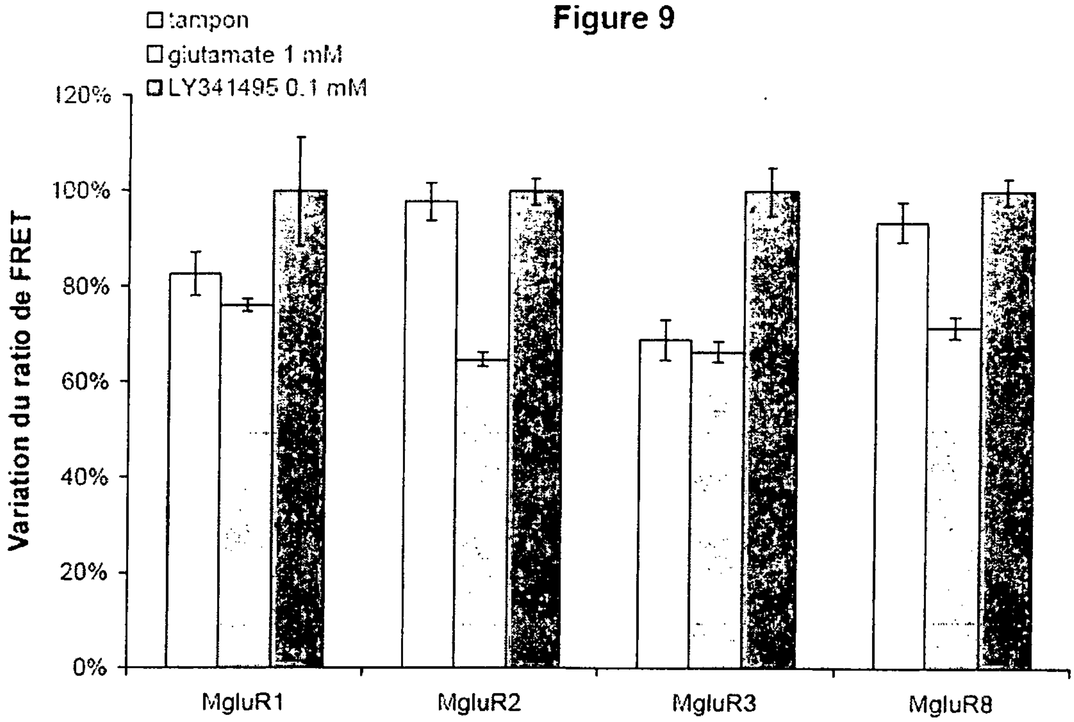


Figure 10

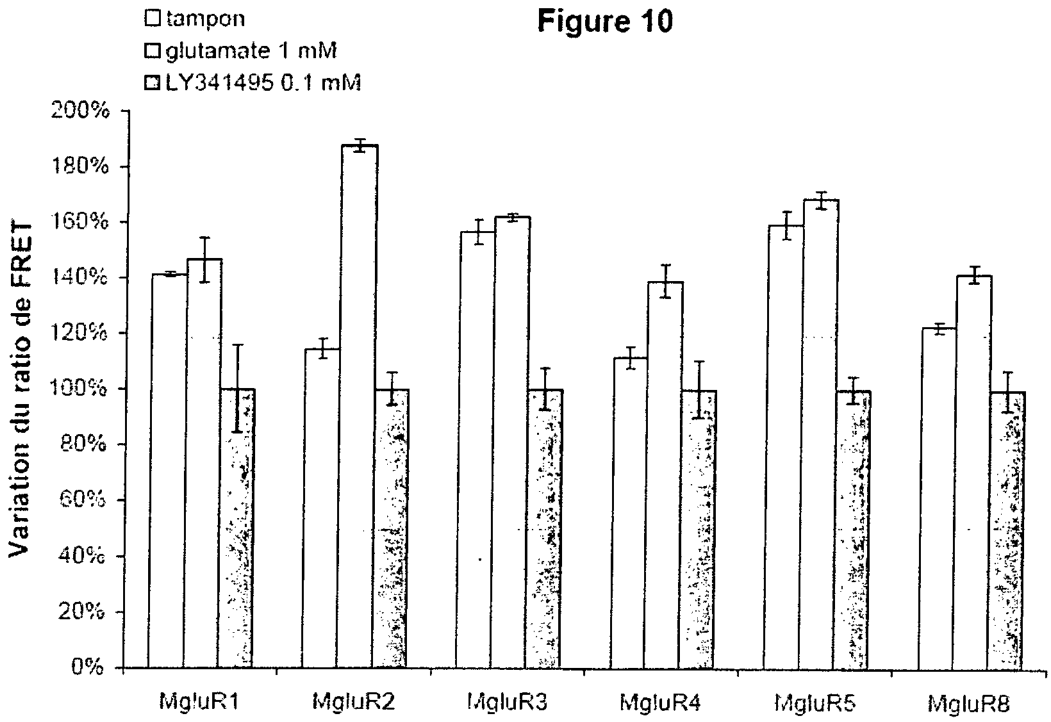

Figure 11

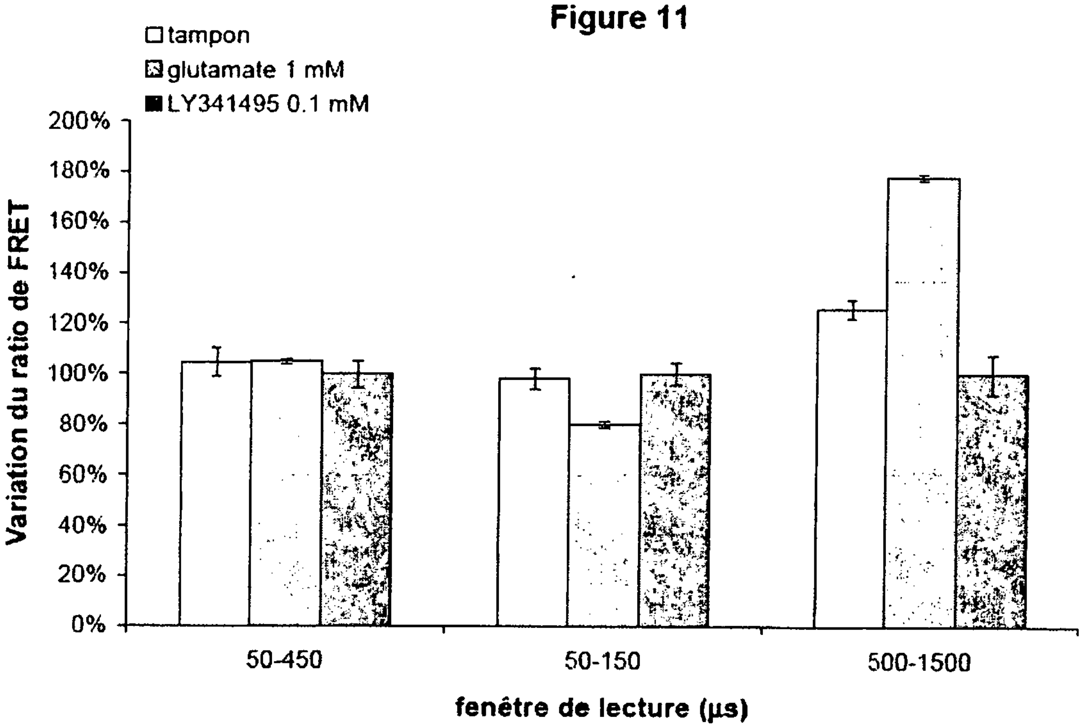


Figure 12

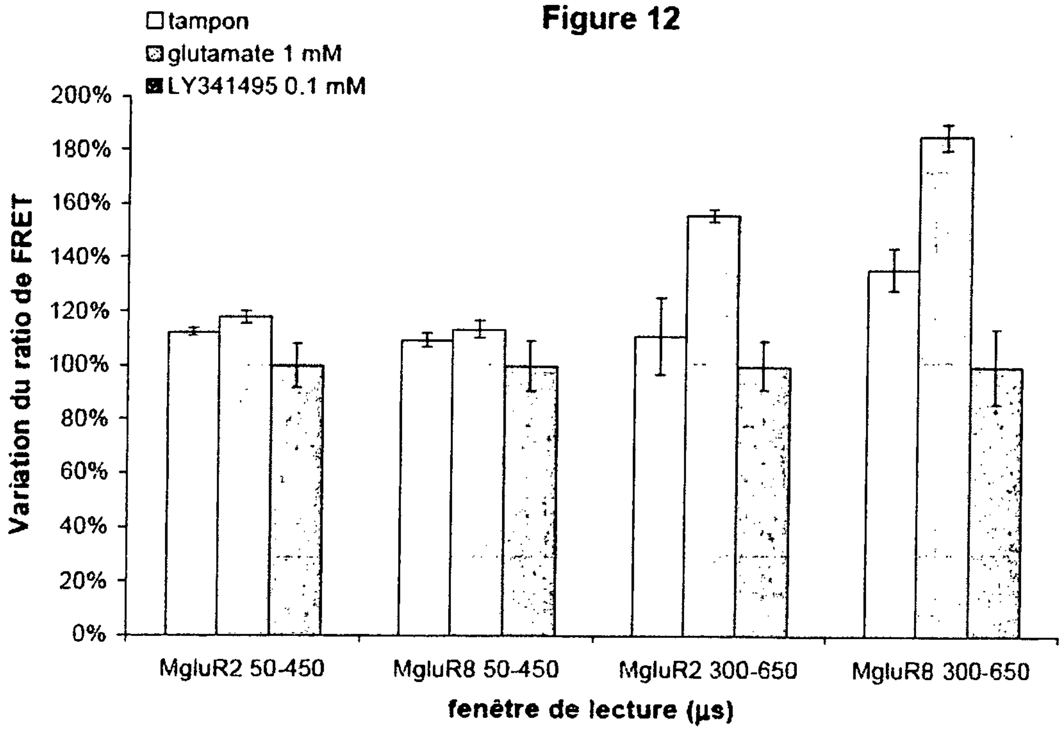

Figure 13A

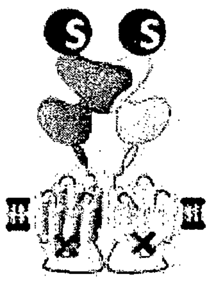


Figure 13B

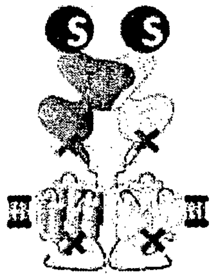


Figure 13C

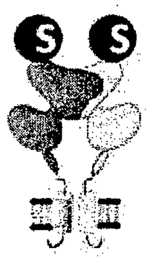


Figure 13D

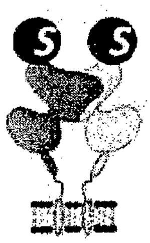


Figure 13E

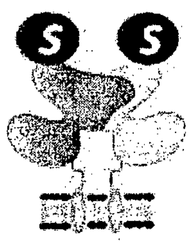


Figure 13F

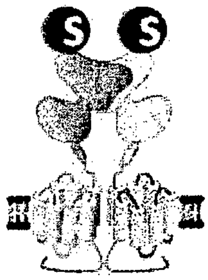

**Figure 14**

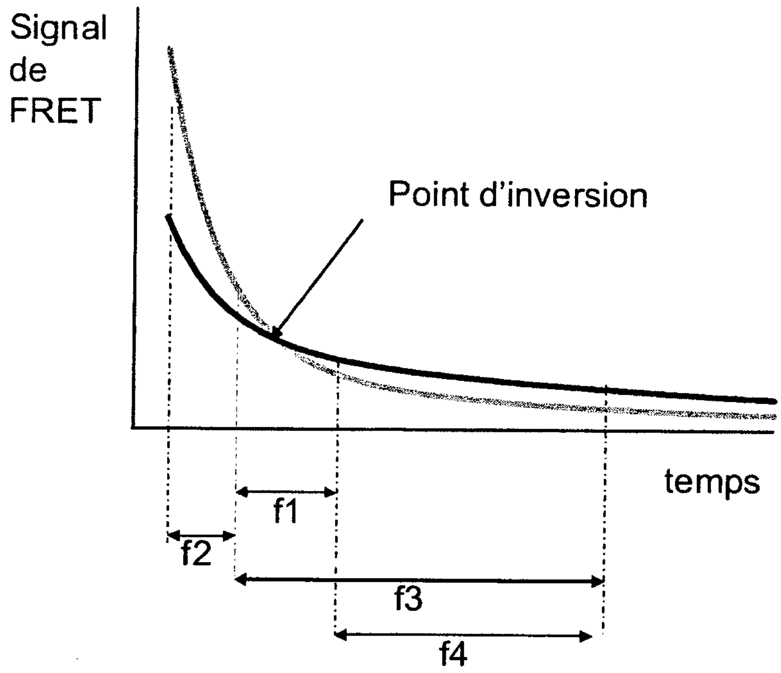

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6824990 B
- WO 2007026099 A
- EP 0180492 A
- EP 0321353 A
- EP 0601113 A
- WO 0196877 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 0403593 A
- US 5324825 A
- US 5202423 A
- US 5316909 A
- WO 200910580 A
- WO 2008063721 A
- US 5622821 A
- WO 04072232 A2
- US 6800651 B


### Littérature non-brevet citée dans la description


- **KUNISHIMA et al.** *Nature,* 26 Octobre 2000, vol. 407 (6807), 971-7
- **TSUCHIYA et al.** *Proc Natl Acad Sci U S A.,* 05 Mars 2002, vol. 99 (5), 2660-5
- **MAUREL et al.** *Nat. Methods,* Juin 2008, vol. 5 (6), 561-7
- **FERRAGUTI et al.** *Cell Tissue Res.,* Novembre 2006, vol. 326 (2), 483-504
- **MALITSCHEK et al.** *Mol Pharmacol.,* Août 1999, vol. 56 (2), 448-54
- **LIU et al.** *J Biol Chem.,* 16 Avril 2004, vol. 279 (16), 15824-3
- **GONZÀLEZ-MAESO et al.** *Nature,* Mars 2008, vol. 452, 93-99
- **MAUREL et al.** *Nature methods,* 2008
- **POOLE R. et al.** Synthesis and characterisation of highly emissive and kinetically stable lanthanide complexes suitable for usage in cellulo. *dans Biomol. Chem,* 2005, vol. 3, 1013-1024
- **B.A.GRIFFIN et al.** *Science,* 1998, vol. 281, 269-271
- **S.A. ADAMS et al.** *J. Am. Chem. Soc.,* 2002, vol. 124, 6063-6076
- **C. M. MCCANN et al.** *Biotechnique,* 2005, vol. 38, 945-952
- **JUILLERAT et al.** *Chemistry & biology,* Avril 2003, vol. 10, 313-317
- **GAUTIER et al.** *Chemistry et Biology,* Février 2008, vol. 15, 128-136
- **GRONEMEYER et al.** *Protein engineering, design & selection,* 2006, vol. 19 (7), 309-3016
- **N.GEORGE et al.** *Journal of the American Chemical society,* 2004, vol. 126, 8896-8897
- *Nature Methods,* 2008